# EUROPÄISCHE PATENTSCHRIFT

(11) **EP 0 451 103 B1**
(45) Veröffentlichungstag und Bekanntmachung des Hinweises auf die Patenterteilung: **13.07.1994**
(21) Anmeldenummer: 91810217.9
(22) Anmeldetag: 26.03.1991
(51) Int. Cl.: A61K 9/127, A61K 31/40

(54) **Parenteral applizierbare Liposomenformulierung aus synthetischen Lipiden**
Parenterally applicable liposome formulation of synthetic lipids
Formulation pour application parentérale de liposomes à base de lipides synthétiques

(30) Priorität: 03.04.1990 CH 1104/90
(43) Veröffentlichungstag der Anmeldung: 09.10.1991
(73) Patentinhaber: CIBA-GEIGY AG, 4002 Basel (CH)
(72) Erfinder: Schneider, Peter, Dr., CH-4103 Bottmingen (CH); van Hoogevest, Peter, Dr., CH-4125 Riehen (CH); Capraro, Hans Georg, Dr., CH-4310 Rheinfelden (CH); Isele, Ute, W-7841 Bad Bellingen (DE)

(56) Entgegenhaltungen:
- EP-A- 0 178 624
- WO-A-88/06441
- CHEMICAL ABSTRACTS, Band 108, Nr. 11, 14. März 1988, Seite 339, ZusammenfassungNr. 91072n, Columbus, Ohio, US; E. REDDI et al.: "Pharmacokinetic studies withzinc(II)-phthalocyanine in tumor-hearing mice",& BR. J. CANCER 1987, 56(5), 597-600 (Kat. D,Y)
- CHEMICAL ABSTRACTS, Band 112, Nr. 26, 25. Juni 1990, Seiten 321-322,Zusammenfassung Nr. 240366y, Columbus, Ohio, US; F. GINEVRA et al.: "Deliveryof the tumor photosensitizer zinc(II)-phthalocyanine to serum proteins bydifferent liposomes: studies in vitro and in vivo",& CANCER LETT. (SHANNON, IREL.) 1990, 49(1), 59-65 (Kat. Y)
- CHEMICAL ABSTRACTS, Band 113, Nr. 6, 6. August 1990, Seiten 346-347,Zusammenfassung Nr. 46168r, Columbus, Ohio, US; E. REDDI et al.: "Liposome- orLDL-adminstered zinc (II)-phthalocyanine as a photodynamic agent for tumors. I.Pharmacokinetic properties and phototherapeutic efficiency", & BR.J. CANCER 1990, 61(3), 407-11 (Kat. Y)
- J. Inorg. Biochem. 29, 59-65 (1987)

## Beschreibung

Gegenstand der Erfindung sind pharmazeutische Zusammensetzungen in Form von parenteral applizierbaren Liposomendispersionen oder dafür verwendbaren Trockenpräparaten enthaltend den Zink-Phthalocyaninkomplex und synthetische, im wesentlichen reine Phospholipide, ein neues erfinderisches Verfahren zur Herstellung dieser pharmazeutischen Zusammensetzungen sowie die Verwendung der Trockenpräparate zur Herstellung von intravenös applizierbaren Liposomendispersionen bzw. die Verwendung des Zink-Phthalocyaninkomplexes und der synthetischen, im wesentlichen reinen Phospholipide zur Herstellung von Trockenpräparaten. Ebenfalls Gegenstand der Erfindung ist die Anwendung der pharmazeutischen Zusammensetzungen in einem Verfahren zur therapeutischen Behandlung des menschlichen oder tierischen Körpers.

Sowohl der Zink-Phthalocyaninkomplex selbst als auch dessen therapeutische Verwendung in der photodynamischen Chemotherapie bei der Behandlung von Tumoren, siehe J.D. Spikes, Photochem. Photobiol. 43, 691 (1986), sind bekannt. Der Zink-Phthalocyaninkomplex wird als wässrige Suspension in-vivo an Mäuse oder Ratten intraperitoneal verabreicht und das an den Versuchstieren generierte Karcinom mit energiereichem Licht, vorzugsweise mit gebündeltem sichtbarem Licht (LASER), bestrahlt.

Für die humantherapeutische Verwendung sind intraperitoneale Applikationsformen wegen der auftretenden Schmerzen beim Einstich in die Bauchhöhle und der hohen Anforderungen an die Geschicklichkeit des Arztes generell problematisch. Es wird daher eine alternative parenterale Applikationsform mit verbesserter Akzeptanz durch den Patienten angestrebt, die ebenfalls eine systemische Verteilung des zu applizierenden Zink-Phthalocyaninkomplexes gewährleisten kann.

Die intravenöse Applikationsform bietet eine systemische Verteilung des Wirkstoffes. Sie setzt aber dessen homogene Verteilung in der wässrigen Injektionsflüssigkeit voraus.

Zur Herstellung von geeigneten intravenösen Applikationsformen ist daher vorgeschlagen worden, statt des schwerlöslichen Zink-Phthalocyaninkomplexes dessen wasserlösliche Derivate zu verwenden und diese intravenös zu applizieren, siehe J. Rousseau et al. Int. J. Appl. Radiat. Isot. 36, 709 (1985).

Das Einführen von hydrophilen Gruppen wie Sulfonylgruppen in das Porphyringerüst des Zink-Phthalocyaninkomplexes erhöht zwar die Wasserlöslichkeit dieses Komplexes. Allerdings werden für die pharmazeutische Verwendung unbrauchbare Derivate mit fehlendem Standard erhalten, da diese aus uneinheitlichen Gemischen der mono- bis tetrasubstituierten Derivate mit mehreren Regioisomeren bestehen, siehe F.H. Moser et al., The Phthalocyanines, CRC Press, 1983, Vol. II, Page 20. Die Trennung der zahlreichen isomeren Derivate wäre mit unrealistischem Aufwand verbunden.

Alternativ hat man vorgeschlagen, den chemisch reinen, wasserunlöslichen Zink-Phthalocyaninkomplex in wässriger Phase durch Zusatz eines Vehikels zu solubilisieren. So lässt sich mit geeigneten Löslichkeitsvermittlern in Form von Phospholipiden, z.B. Dipalmitoylphosphatidylcholin, der Komplex in unilamellaren Liposomen verkapseln, die in wässriger Phase weitgehend homogen dispergierbar sind, siehe E. Reddi et al., Br. J. Cancer (1987), 56, Seiten 597-600.

Diese homogene Liposomendispersion ist für die Zwecke der intravenösen Applikation am Menschen trotzdem noch ungeeignet, da die Dispersion nach der sogenannten Injektionsmethode unter Verwendung grösserer Mengen des toxischen Pyridins hergestellt wird, siehe G. Valduga et al., J. Inorg. Biochem. 29, 59-65 (1987). Pyridin gehört zu den wenigen Lösungsmitteln, worin der Zinkphthalocyaninkomplex sich überhaupt löst. Man verdünnt diese Lösung mit Äthanol und injiziert die Pyridin-haltige äthanolische Lösung bei erhöhter Temperatur in Wasser oder Pufferlösung. Nach dieser Methode wird wegen Azeotropbildung stets ein prozentualer Anteil des toxischen Lösungsmittels Pyridin in der wässrigen Phase zurückbleiben.

Auch die Herstellung von Liposomendispersionen nach anderen Verfahren ohne die Verwendung von organischen Lösungsmitteln ist problematisch. Selbst wenn man lösungsmittelfreie Trockenpräparate wie Lyophilisate oder Eindampfrückstände für die Bildung der wässrigen Liposomendispersionen verwendet, kann die Herstellung dieser Trockenpräparate seinerseits wegen der Lipophilie und Wasserunlöslichkeit der Lipidkomponenten nur aus Lösungen in ausgewählten organischen Lösungsmitteln oder Lösungsmittelgemischen erfolgen. In diesen Lösungsmitteln müssen sowohl der Zink-Phthalocyaninkomplex als auch die verwendeten Phospholipide vollständig löslich sein. Beim Verdampfen der Lösungsmittel muss ein homogenes und rieselfähiges Pulver entstehen. Es darf keine Entmischung der Komponenten stattfinden. Dies hätte zur Folge, dass das Trockenpräparat verklebt und sich keine Liposomen, sondern nur schlecht dispersionsfähige Aggregate bilden, z.B. grosse Mizellen, welche Embolien verursachen können.

Aus der EP-A-178624 sind Trockenpräparate und daraus herstellbare Liposomendispersionen bekannt, welche aus synthetischen, im wesentlichen reinen Phospholipiden bestehen und mit verkapselten pharmazeutischen Wirkstoffen in der Chemotherapie von Tumoren verwendbar sind.

Der vorliegenden Erfindung liegt die Aufgabe zugrunde, eine parenteral, insbesondere intravenös, applizierbare Liposomendispersion unter Verwendung solcher Lösungsmittel herzustellen, welche den Zink-Phthalocyaninkomplex und die Phospholipidkomponenten vollständig lösen und deren Restmenge aus nicht mehr entfernbaren Lösungsmittelrückständen weniger als 1 % beträgt, welche für intravenöse Formulierungen toxikologisch unbedenklich wäre.

Die Erfindung betrifft pharmazeutische Zusammensetzungen in Form von parenteral applizierbaren Liposomendispersionen enthaltend
a) den Zink-Phthalocyaninkomplex,
b) ein synthetisches, zu mehr als 90 % reines Phospholipid der Formel 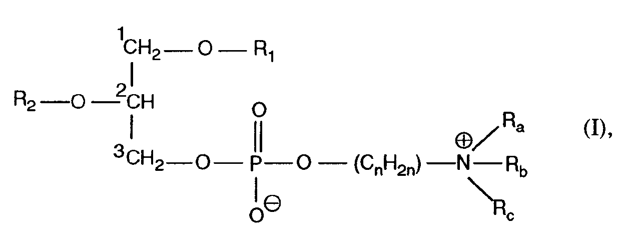 worin R₁ C₁₀-C₂₀-Alkanoyl mit gerader Anzahl an C-Atomen, R₂ C₁₀-C₂₀-Alkenoyl mit gerader Anzahl an C-Atomen, Rₐ, R_{b} und R_{c} Wasserstoff oder C₁-C₄-Alkyl und n eine ganze Zahl von zwei bis vier darstellen, gegebenenfalls kombiniert mit einem
c) synthetischen, zu mehr als 90 % reinen Phospholipid der Formel 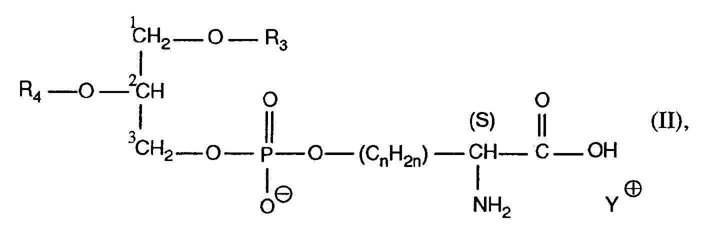 worin R₃ und R₄ unabhängig voneinander C₁₀-C₂₀-Alkenoyl mit gerader Anzahl an C-Atomen, n eine ganze Zahl von eins bis drei und Y^{⊕} das Kation einer pharmazeutisch annehmbaren Base darstellen, und
d) eine pharmazeutisch annehmbare Trägerflüssigkeit und gegebenenfalls für parenterale Darreichungsformen geeignete wasserlösliche Hilfsstoffe.

Die weiter vorn und im folgenden genannten Begriffe und Definitionen haben im Rahmen der Bescheibung der Erfindung vorzugsweise die folgenden Bedeutungen:

Der Begriff pharmazeutische Zusammensetzung definiert ein Stoffgemisch, das sich zur parenteralen Applikation, im vorliegenden Fall insbesondere zur intravenösen Applikation, an Mensch und Tier, bevorzugt am Menschen, eignet und zur Behandlung von diversen Krankheiten, im vorliegenden Fall von Tumoren, verwendbar ist.

Die parenteral applizierbare Liposomendispersion enthält Liposomen in Form von unilamellaren, multilamellaren, grossen und kleinen Liposomen bestehend aus einer Doppelschichtanordnung der Phospholipide (I) und gegebenenfalls (II) mit einem Innenraum und sphärischer Gestalt (unilamellar) oder bestehend aus mehreren konzentrischen Doppelschichtanordnungen der Phospholipide (I) und gegebenenfalls (II) mit einem Innenraum und sphärischer Gestalt ("zwiebelschalenförmiger" Aufbau der Doppelschichten oder Membranen - multilamellar). Die Grössenordnung der Liposomen ist abhängig vom Herstellungsverfahren variabel zwischen ca. 1,0 x 10⁻⁸ bis ca. 1,0 x 10⁻⁵ m.

Die therapeutische Verwendung von Liposomen als Träger von Wirkstoffen unterschiedlicher Art ist bekannt. So sind Liposomen als Träger von Proteinen, z.B. Antikörpern oder Enzymen, Hormonen, Vitaminen oder Genen oder zu analytischen Zwecken als Träger von markierten Verbindungen vorgeschlagen worden.

Pharmazeutische Darreichungsformen auf Liposomenbasis sind in dem Übersichtswerk von Gregoriadis G. (Herausgeber) Liposome Technology, Vol. II, Incorporation of Drugs, Proteins and Genetic Material, CRC Press 1984, beschrieben. Im Übersichtswerk von Knight, C.G. (Herausgeber), Liposomes: From Physical Structure to Therapeutic Applications, Elsevier 1981, sind im Kapitel 16 auf S. 166 die Vorteile einer pharmazeutischen Darreichungsform auf Liposomenbasis zusammengefasst.

Die Liposomendispersion gemäss vorliegender Erfindung ist frei von Feststoffpartikeln und grösseren Lipidaggregaten, auch bei Zimmertemperatur mehrere Tage bis Wochen lagerstabil, reproduzierbar bezüglich des Mengenanteils der Komponenten, bezüglich der verwendeten Lipidkomponenten und der Restmengen an organischen Lösungsmitteln toxikologisch unbedenklich und aufgrund von in-vitro und in-vivo Befunden für die parenterale, insbesondere intravenöse Applikation am Menschen geeignet.

Der im Zusammenhang mit organischen Resten, z.B. Niederalkyl, Niederalkylen, Niederalkoxy, Niederalkanoyl etc., verwendete Ausdruck "Nieder" bedeutet, dass solche organischen Reste, falls nicht ausdrücklich anders definiert, bis einschliesslich 7 und bevorzugt bis einschliesslich 4 Kohlenstoffatome enthalten.

Die Nomenklatur der Phospholipide der Formeln I und II und die Bezifferung der C-Atome erfolgt anhand der in Eur. J. of Biochem. 79, 11-21 (1977) "Nomenclature of Lipids" von der IUPAC-IUB Commission on Biochemical Nomenclature (CBN) gegebenen Empfehlungen (sn-Nomenklatur, stereospecific numbering).

Der Zink-Phthalocyaninkomplex a) entspricht der in der Publikation von G. Valduga et al. Photochem. and Photobiology Vol. 48, No. 1 (1988), Seiten 1-5, auf der Seite 1 beschriebenen Verbindung, welche seit langem bekannt ist.

Die Reinheit der synthetischen Phospholipide der Formeln I und II beträgt vorzugsweise mehr als 95 Gew.-%.

Dieser Reinheitsgrad ist anhand der bekannten analytischen Methoden, z.B. chromatographisch, wie z.B. HPLC, gaschromatographisch oder papierchromatographisch, nachweisbar.

In einem Phospholipid der Formel I ist R₁ mit der Bedeutung "C₁₀-C₂₀-Alkanoyl mit gerader Anzahl an C-Atomen" vorzugsweise n-Dodecanoyl, n-Tetradecanoyl, n-Hexadecanoyl, n-Octadecanoyl oder n-Icosanoyl.

In einem Phospholipid der Formel I ist R₂ mit der Bedeutung "C₁₀-₂₀-Alkenoyl mit gerader Anzahl an C-Atomen" vorzugsweise 9-cis-Dodecenoyl, 9-cis-Tetradecenoyl, 9-cis-Hexadecenoyl, 6-cis-Octadecenoyl, 6-trans-Octadecenoyl, 9-cis-Octadecenoyl, 9-trans-Octadecenoyl, 11-cis-Octadecenoyl oder 9-cis-Icosenoyl.

In einem Phospholipid der Formel I sind Rₐ, R_{b} und R_{c} vorzugsweise C₁-C₄-Alkyl, insbesondere Methyl.

In einem Phospholipid der Formel I ist n eine ganze Zahl von zwei bis vier, vorzugsweise zwei. Die Gruppe der Formel -(CₙH₂ₙ)- stellt unverzweigtes oder verzweigtes Alkylen dar, z.B. 1,1-Aethylen, 1,1-, 1,2- oder 1,3-Propylen oder 1,2-, 1,3- oder 1,4-Butylen. Bevorzugt ist 1,2-Aethylen (n=2).

In einem besonders bevorzugten Phospholipid der Formel I bedeuten R₁ n-Dodecanoyl, n-Tetradecanoyl, n-Hexadecanoyl oder n-Octadecanoyl und R₂ 9-cis-Dodecenoyl, 9-cis-Tetradecenoyl, 9-cis-Hexadecenoyl, 9-cis-Octadecenoyl oder 9-cis-Icosenoyl. Rₐ, R_{b} und R_{c} sind Methyl und n ist zwei.

Ein ganz besonders bevorzugtes Phospholipid der Formel I ist synthetisches 1-n-Hexadecanoyl-2-(9-cis-octadecenoyl)-3-sn-phosphatidylcholin mit einer Reinheit von mehr als 95 %.

In einem Phosholipid der Formel II haben R₃ und R₄ die unter Formel I für R₁ und R₂ genannten Bedeutungen.

In einem Phospholipid der Formel II sind R₃ und R₄ mit der Bedeutung "C₁₀-C₂₀-Alkenoyl mit gerader Anzahl an C-Atomen" vorzugsweise 9-cis-Dodecenoyl, 9-cis-Tetradecenoyl, 9-cis-Hexadecenoyl, 6-cis-Octadecenoyl, 6-trans-Octadecenoyl, 9-cis-Octadecenoyl, 9-trans-Octadecenoyl, 11-cis-Octadecenoyl oder 9-cis-Icosenoyl.

Das Kation Y^{⊕} einer pharmazeutisch annehmbaren Base ist beispielsweise ein Alkalimetall-, z.B. das Lithium-, Natrium- oder Kaliumion, das Ammoniumion, ein Mono-, Di- oder Tri-C₁-C₄-alkylammoniumion, z.B. das Trimethyl-, Aethyl-, Diäthyl- oder Triäthylammoniumion, das Tetramethylammoniumion, ein 2-Hydroxyäthyl-tri-C₁-C₄-alkylammoniumion, z.B. das Cholinkation, oder das 2-Hydroxyäthylammoniumion, sowie das Kation einer basischen Aminosäure, z.B. Lysin oder Arginin.

Y^{⊕} ist bevorzugt das Natriumion.

In einem besonders bevorzugten Phospholipid der Formel II haben R₃ und R₄ identische Bedeutungen, z.B. 9-cis-Dodecenoyl, 9-cis-Tetradecenoyl, 9-cis-Hexadecenoyl, 9-cis-Octadecenoyl oder 9-cis-Icosenoyl. n ist eins und Y^{⊕} das Natriumion.

Ein ganz besonders bevorzugtes Phospholipid der Formel II ist synthetisches Natrium-1,2-di-(9-cis-octadecenoyl)-3-sn-phosphatidyl-S-serin mit einer Reinheit von mehr als 95 %.

Für die Acylreste in den Phospholipiden der Formel I und II sind auch die in Klammern angegebenen Bezeichnungen gebräuchlich:
9-cis-Dodecenoyl (Lauroleoyl), 9-cis-Tetradecenoyl (Myristoleoyl), 9-cis-Hexadecenoyl (Palmitoleoyl), 6-cis-Octadecenoyl (Petroseloyl), 6-trans-Octadecenoyl (Petroselaidoyl), 9-cis-Octadecenoyl (Oleoyl), 9-trans-Octadecenoyl (Elaidoyl), 11-cis-Octadecenoyl (Vaccenoyl), 9-cis-Icosenoyl (Gadoleoyl), n-Dodecanoyl (Lauroyl), n-Tetradecanoyl (Myristoyl), n-Hexadecanoyl (Palmitoyl), n-Octadecanoyl (Stearoyl), n-Icosanoyl (Arachidoyl).

In der pharmazeutisch annehmbaren Trägerflüssigkeit d) sind die Komponenten a) und b) oder a), b) und c) als Liposomen, vorzugsweise multilamellare Liposomen, so enthalten, dass sich mehrere Tage bis Wochen keine Feststoffe oder feste Aggregate wie Mizellen zurückbilden und die klare oder gegebenenfalls leicht opaleszierende Flüssigkeit mit den genannten Komponenten, gegebenenfalls nach Filtration, parenteral, vorzugsweise intravenös, applizierbar ist.

In der Trägerflüssigkeit d) können z.B. pharmazeutisch annehmbare, nicht toxische wasserlösliche Hilfsstoffe enthalten sein, welche zur Herstellung von isotonischen Bedingungen erforderlich sind, z.B. ionische Zusätze wie Kochsalz oder nichtionische Zusätze (Gerüstbildner) wie Sorbit, Mannit, Glucose oder Lactose. Insbesondere sind diese Zusätze, z.B. Kochsalz oder Mannit, im Trockenpräparat in den vorgeschriebenen Mengen enthalten, welche zur Herstellung von isotonischen Bedingungen der parenteral applizierbaren Lösungen erforderlich sind.

Geeignete wasserlösliche Hilfsstoffe in der Lösung und im Trockenpräparat sind ausserdem für flüssige pharmazeutische Formulierungen verwendbare Netzmittel oder Tenside im eigentlichen Sinn, insbesondere nichtionische Tenside vom Typ Fettsäure-Polyhydroxyalkoholester wie Sorbitanmonolaurat, -oleat, -stearat oder -palmitat, Sorbitantristearat oder -trioleat, Polyoxyethylen-Addukte von Fettsäure-Polyhydroxyalkoholestern wie Polyoxyethylen-sorbitanmonolaurat, -oleat, -stearat, -palmitat, -tristearat oder -trioleat, Polyethylenglycol-Fettsäureester wie Polyoxyethylstearat, Polyethylenglycol-400-stearat, Polyethylenglycol-2000-stearat, insbesondere Ethylenoxid-Propylenoxid Blockpolymere vom Typ Pluronic® (Wyandotte Chem. Corp.) oder Synperonic® (ICI).

Die Liposomendispersion ist aus einem Trockenpräparat herstellbar, welches ebenfalls Gegenstand der vorliegenden Erfindung ist.

Die vorliegende Erfindung betrifft ebenfalls pharmazeutische Zusammensetzungen in Form eines Trockenpräparats enthaltend
a) den Zink-Phthalocyaninkomplex,
b) ein synthetisches, zu mehr als 95 % reines Phospholipid der Formel I worin R₁, R₂, Rₐ, R_{b} und R_{c} sowie n die genannten Bedeutungen haben, gegebenenfalls kombiniert mit einem
c) synthetischen, zu mehr als 95 % reinen Phospholipid der Formel II, worin R₃, R₄, n und Y^{⊕} die genannten Bedeutungen haben, und gegebenenfalls
d) für intravenöse Darreichungsformen geeignete, wasserlösliche Hilfsstoffe.

Ein Trockenpräparat ist der nach einem beliebigen thermischen Trocknungsverfahren wie Verdampfen bei Raumtemperatur oder erhöhter Temperatur oder vorzugsweise Gefriertrocknung erhältliche Rückstand, welcher weniger als 1 % (Gew.) vorzugsweise weniger als 0,5 %, organische Lösungsmittelreste wie Piperidin, tert-Butanol oder Dimethylsulfoxid enthält.

In dem Trockenpräparat ist der Zink-Phthalocyaninkomplex in einem Mengenverhältnis von ca. 0,1 bis 5,0 Gew.-%, vorzugsweise 0,1-1,0 Gew.-%, bezogen auf die Gesamtmenge der Phospholipide der Formeln I und gegebenenfalls II - Komponenten b) und gegebenenfalls c) - enthalten. Im Trockenpräparat beträgt das Mischungsverhältnis der Phospholipide der Formel I - Lecithinkomponente - zu Phospholipiden der Formel II - Serinkomponente - ca. 50 zu 50 Gew.-%, vorzugsweise 70 zu 30 Gew.-%.

Das Trockenpräparat der vorliegenden Erfindung zeichnet sich durch gute Abfüllbarkeit, exakte Reproduzierbarkeit der Einwaagen der Dosiseinheitsformen und Lagerstabilität aus.

Die vorliegende Erfindung betrifft ebenfalls ein Verfahren zur Herstellung einer pharmazeutischen Zusammensetzung in Form einer parenteral applizierbaren Liposomendispersion, welche dadurch gekennzeichnet ist, dass man ein Trockenpräparat enthaltend:
a) den Zink-Phthalocyaninkomplex,
b) ein synthetisches, zu mehr als 90 % reines Phospholipid der Formel I, worin R₁, R₂, Rₐ, R_{b} und R_{c} und n die genannten Bedeutungen haben, gegebenenfalls kombiniert mit einem
c) synthetischen, zu mehr als 90 % reinen Phospholipid der Formel II, worin R₃, R₄, n und Y^{⊕} die genannten Bedeutungen haben, und gegebenenfalls
d) für parenterale Darreichungsformen geeignete, wasserlösliche Hilfsstoffe in wässriger Phase dispergiert und gegebenenfalls die erhältliche wässrige Dispersion auf pH 7,0-7,8 abpuffert und/oder eine Fraktion von Liposomen mit einem gewünschten Bereich des Durchmessers isoliert.

Die einzelnen Schritte des Verfahrens werden in an sich bekannter Weise so duchgeführt, dass man das erfindungsgemäss erhältliche Trockenpräparat vor der Applikation als Liposomendispersion in der vorgesehenen Flüssigkeitsmenge, insbesondere in keimfreiem Wasser (pyrogenfrei) zur Injektion, rekonstituiert.

Man dispergiert beispielsweise durch Schütteln (z.B. Vortex-Mischer) oder Rühren der wässrigen Phase, der man zuvor das Trockenpräparat zugesetzt hat. Die Bildung von Liposomen, welche gross, klein, unilamellar oder multilamellar sein können, findet spontan, d.h. ohne zusätzliche Energiezufuhr von aussen und mit grosser Geschwindigkeit statt. Es können ca. 0,1 bis 50 Gewichtsprozent (bezogen auf das Gesamtgewicht der wässrigen Dispersion), vorzugsweise 2 bis 20 Gewichtsprozent, des Trockenpräparates in wässriger Phase dispergiert werden.

Man puffert vorzugsweise sauer oder basisch reagierende wässrige Dispersionen auf pH 7,0-7,8, vorzugsweise pH 7,2-7,4, ab. Gegebenenfalls dispergiert man in einer bereits auf diesen pH-Wert abgepufferten, wässrigen Pufferlösung.

Man dispergiert bei Temperaturen unterhalb ca. 36°C, vorzugsweise bei Raumtemperatur. Gegebenenfalls führt man das Verfahren unter Kühlen und/oder Inertgasatmosphäre, z.B. Stickstoff- oder Argonatmosphäre, durch. Die erhältlichen Liposomen sind in wässriger Phase sehr lange (bis zu mehrere Wochen oder Monate) beständig.

Die Grösse der gebildeten Liposomen ist u.a. von der Menge des Wirkstoffs und der Lipidkomponenten, deren Mischungsverhältnis und der Konzentration der Komponenten in der wässrigen Dispersion und der Herstellungsmethode abhängig. So kann man beispielsweise durch Erhöhung oder Erniedrigung der Konzentration der Lipidkomponenten wässrige Phasen mit einem hohen Anteil an kleinen oder grossen Liposomen herstellen.

Durch Nachbehandlung der Liposomendispersion, z.B. durch Beschallung mit Ultraschall oder Extrusion durch geradporige Filter (z.B. Nucleopore®) kann man eine besonders einheitliche Grössenverteilung der Liposomen erhalten.

Die Trennung und Isolierung einer Fraktion von grossen Liposomen von einer Fraktion mit kleinen Liposomen, sofern überhaupt notwendig, erfolgt mittels herkömmlicher Trennmethoden, z.B. Gelfiltration, z.B. mit Sepharose® 4B oder Sephacryl® (Pharmacia SE) als Träger, oder durch Sedimentation der Liposomen in der Ultrazentrifuge, z.B. mit einem Schwerefeld bei 160,000 x g. Beispielsweise setzen sich nach mehrstündigem, z.B. ca. dreistündigem, Zentrifugieren in diesem Schwerefeld grössere Liposomen ab, während kleinere Liposomen dispergiert bleiben und dekantiert werden können. Nach mehrmaligem Zentrifugieren erreicht man eine vollständige Trennung der grossen von den kleinen Liposomen.

Insbesondere durch Gelfiltration kann man alle in der wässrigen Phase befindlichen Liposomen mit einem Durchmesser grösser als ca. 6,0 x 10⁻⁸ m sowie nicht verkapselte Komponenten und überschüssige, dispergierte Lipide, welche in hochmolekularen Aggregaten vorliegen, abtrennen und so eine wässrige Dispersion mit einer Fraktion von Liposomen mit relativ einheitlicher Grösse herstellen.

Die erfolgte Bildung von Liposomen und ihr Gehalt in wässriger Phase lässt sich in an sich bekannter Weise anhand verschiedener physikalischer Messmethoden nachweisen, z.B. mit gefriergebrochenen (freeze fracture) Proben und Dünnschnitten im Elektronenmikroskop oder durch Röntgendiffraktion, durch dynamische Lichtstreuung, durch Massenbestimmung des Filtrates in der analytischen Ultrazentrifuge und vor allem spektroskopisch, z.B. im Kernresonanzspektrum (¹H, ¹³C und ³¹P).

Gegenstand der vorliegenden Erfindung ist ebenfalls ein neues, erfinderisches Verfahren zur Herstellung einer pharmazeutischen Zusammensetzung in Form eines Trockenpräparats, welches dadurch gekennzeichnet ist, dass man
a) den Zink-Phthalocyaninkomplex und
b) ein synthetisches, zu mehr als 90 % reines Phospholipid der Formel I, worin R₁, R₂, Rₐ, R_{b} und R_{c} und n die genannten Bedeutungen haben, gegebenenfalls kombiniert mit einem
c) synthetischen, zu mehr als 90 % reinen Phospholipid der Formel II, worin R₃ und R₄, n und Y^{⊕} die genannten Bedeutungen haben, in einem pharmazeutisch annehmbaren organischen Lösungsmittel löst, dessen Restmenge in einem Trockenpräparat toxikologisch unbedenklich ist, und gegebenenfalls
d) Trägerflüssigkeit und für parenterale Darreichungsformen geeignete wasserlösliche Hilfsstoffe zusetzt und das Lösungsmittel oder Lösungsmittelgemisch entfernt.

Ein pharmazeutisch annehmbares organisches Lösungsmittel, dessen Restmenge in einem Trockenpräparat toxikologisch unbedenklich ist, eignet sich zur Herstellung einer klaren Lösung des Zink-Phthalocyaninkomplexes und gegebenenfalls der Phospholipid-Komponenten (I) und (II). Sind die Phospholipide in dem organischen Lösungsmittel, worin der Zink-Phthalocyaninkomplex löslich ist, nicht löslich, werden diese in einem zweiten Lösungsmittel, z.B. in tert-Butanol, gelöst und diese Lösung mit der Lösung des Zink-Phthalocyaninkomplexes vereinigt.

Bevorzugte organische Lösungsmittel, welche die toxikologischen Anforderungen an die im Trockenpräparat vorhandenen Restmengen erfüllen und worin der Zink-Phthalocyaninkomplex löslich ist, sind Dimethylsulfoxid, N-Methyl-2-pyrrolidon und Piperidin sowie Gemische davon.

In Dimethylsulfoxid und N-Methyl-2-pyrrolidon (NMP) sind nur der Zink-Phthalocyaninkomplex, nicht aber die Phospholipide (I) und (II) löslich. In Piperidin sind sowohl der Zink-Phthalocyaninkomplex als auch die Phospholipide (I) und (II) löslich. Alle Lösungsmittel sind bei den im Trockenpräparat vorhandenen Restmengen von weniger als 1 %, vorzugsweise weniger als 0,5 %, toxikologisch unbedenklich.

Bei Verwendung von Dimethylsulfoxid oder NMP löst man den Zink-Phthalocyaninkomplex in der erforderlichen Mindestmenge dieser Lösungsmittel auf und vereinigt diese Lösung mit einer zweiten Lösung der Phospholipide, welche mit der ersten Lösung des Zink-Phthalocyaninkomplexes mischbar ist. An dieses zweite Lösungsmittel sind neben seiner Mischbarkeit mit der Dimethylsulfoxid-Lösung dieselben toxikologischen Anforderungen an die im Trockenpräparat vorhandenen Restmengen zu stellen. Ein solches geeignetes Lösungsmittel, worin sich die Phospholipide (I) und (II) vollständig lösen, ist z.B. tert-Butanol.

Die Herstellung des Trockenpräparates, vorzugsweise des Lyophilisats, kann durch Abwandlung bekannter Verfahren erfolgen, indem man zunächst die vorgelegte Menge des Phospholipids, z.B. 1-Hexadecanoyl-2-(9-cis-octadecenoyl)-3-sn-phosphatidylcholin, gegebenenfalls unter leichtem Erwärmen, in der für den Lösungsvorgang benötigten Menge tert-Butanol löst und diese klare Lösung mit einer zweiten Lösung enthaltend eine definierte Menge des Zink-Phthalocyaninkomplexes in einer für den Lösungsvorgang bemessenen Menge Dimethylsulfoxid oder NMP vereinigt. Alternativ kann man die Phospholipide (I) und (II) bei ca. 0°C bis Raumtemperatur, bevorzugt bei 0°C, und den Zink-Phthalocyaninkomplex bei Raumtemperatur bis ca. 40°C, bevorzugt bei Raumtemperatur, in der erforderlichen Mindestmenge Piperidin oder in Piperidin enthaltend bis ca. 10 %, vorzugsweise ca. 2-3 %, Wasser lösen und aus dieser klaren Lösung zur Herstellung des Trockenpräparates das Lösungsmittel entfernen. Die klare Lösung in den genannten organischen Lösungsmitteln kann man anschliessend mit der Trägerflüssigkeit d) enthaltend wasserlösliche Hilfsstoffe, insbesondere nicht-ionische Zusätze wie Lactose, versetzen. Das Trockenpräparat kann man durch Entfernen des Lösungsmittelgemischs bei niedriger Temperatur unterhalb ca. 0°C (Lyophilisation) oder bei normaler oder erhitzter Temperatur (Filmbildung) herstellen. Das zur Herstellung des Trockenpräparates verwendete Lösungsmittelgemisch kann man auch zwecks Reinigung dialysieren und die dialysierte wässrige Lösung, welche kein organisches Lösungsmittel enthält, durch Ultrafiltration konzentrieren. Anschliessend stellt man durch Entfernen von Wasser, vorzugsweise durch Lyophilisation, das Trockenpräparat her. Abgemessene Mengen der zu lyophilisierenden Lösung lassen sich in geeignete Behälter für eine Dosiseinheit wie Ampullen, z.B. Glasstechampullen (Vials), abfüllen. Die abgefüllten Behälter kann man anschliessend bei ca. -40° bis -50°C, insbesondere bei -45°C, einfrieren und anschliessend bei einem Druck von ca. 0,2-0,6 mbar durch langsames Erwärmen bis zu einer Endtemperatur von ca. 25°-35°C lyophilisieren.

Das Lösungsmittel oder Lösungsmittelgemisch mit den Komponenten a), b) und c) lässt sich auch direkt ohne Abkühlen thermisch in einem grösseren Gefäss in ein Trockenpräparat überführen (Filmbildungsmethode). Allerdings ist die Gefriertrocknung aus kleinen Gefässen mit einer Dosiseinheit bevorzugt, weil diese Methode einen Abfüllvorgang des Trockenpräparats selbst vermeidet und dadurch die genaue Zuteilung von Dosiseinheiten aus Flüssigkeiten ermöglicht.

Überraschenderweise gelingt es, mit den genannten Verfahren Trockenpräparate, insbesondere Lyophilisate, und daraus rekonstituierbare Liposomendispersionen reproduzierbar herzustellen, welche stabil und zur Injektion geeignet sind.

Die Verwendung des gemäss den genannten Verfahren erhältlichen Trockenpräparates zur Herstellung von intravenös applizierbaren Liposomendispersionen ist ebenfalls Gegenstand der vorliegenden Erfindung.

Die Verwendung des Zink-Phthalocyaninkomplexes und der Phospholipidkomponenten der Formel I und gegebenenfalls der Formel II zur Herstellung von Trockenpräparaten, insbesondere Lypophilisaten, nach der weiter vorn beschriebenen Methodik ist ebenfalls Gegenstand der vorliegenden Erfindung. Ebenfalls Gegenstand der Erfindung ist die Anwendung der pharmazeutischen Zusammensetzungen in einem Verfahren zur therapeutischen Behandlung des menschlichen oder tierischen Körpers, insbesondere bei der Chemotherapie zur Behandlung von Tumoren. Die Liposomendispersion wird parenteral, insbesondere intravenös, verabreicht und das Karcinom mit energiereichem Licht, vorzugsweise mit gebündeltem, sichtbaren Licht (LASER), bestrahlt.

Die Erfindung betrifft vorzugsweise pharmazeutische Zusammensetzungen in Form von intravenös applizierbaren Liposomendispersionen enthaltend
a) den Zink-Phthalocyaninkomplex,
b) ein synthetisches, zu mehr als 95 % reines Phospholipid der Formel I, worin R₁ n-Dodecanoyl, n-Tetradecanoyl, n-Hexadecanoyl oder n-Octadecanoyl und R2 9-cis-Dodecenoyl, 9-cis-Tetradecenoyl, 9-cis-Hexadecenoyl, 9-cis-Octadecenoyl oder 9-cis-Icosenoyl, Rₐ, R_{b}, R_{c} Methyl und n zwei bedeuten, gegebenenfalls kombiniert mit einem
c) synthetischen, zu mehr als 95 % reinen Phospholipid der Formel II, worin R₃ und R₄ identische Bedeutungen haben und 9-cis-Dodecenoyl, 9-cis-Tetradecenoyl, 9-cis-Hexadecenoyl, 9-cis-Octadecenoyl oder 9-cis-Icosenoyl bedeuten, n eins und Y^{⊕} das Natriumion ist, und
d) eine pharmazeutisch annehmbare Trägerflüssigkeit und gegebenenfalls für intravenöse Darreichungsformen geeignete wasserlösliche Hilfsstoffe.

Bevorzugter Erfindungsgegenstand sind ebenfalls pharmazeutische Zusammensetzungen in Form von Trockenpräparaten enthaltend die genannten bevorzugten Komponenten a), b), gegebenenfalls kombiniert mit c), sowie d) für intravenöse Darreichungsformen geeignete, wasserlösliche Hilfsstoffe.

Die Erfindung betrifft insbesondere pharmazeutische Zusammensetzungen in Form von intravenös applizierbaren Liposomendispersionen enthaltend:
a) den Zink-Phthalocyaninkomplex,
b) synthetisches, zu mehr als 95 % reines 1-n-Hexadecanoyl-2-(9-cis-octadecenoyl)-3-sn-phosphatidylcholin (I), gegebenenfalls kombiniert mit
c) synthetischem, zu mehr als 95 % reinen Natrium-1,2-di-(9-cis-octadecenoyl)-3-sn-phosphatidyl-S-serin (II), und
d) eine pharmazeutisch annehmbare Trägerflüssigkeit und gegebenenfalls für intravenöse Darreichungsformen geeignete wasserlösliche Hilfsstoffe.

Besonders bevorzugter Erfindungsgegenstand sind pharmazeutische Zusammensetzungen in Form von Lyophilisaten enthaltend
a) den Zink-Phthalocyaninkomplex,
b) synthetisches, zu mehr als 95 % reines 1-n-Hexadecanoyl-2-(9-cis-octadecenoyl)-3-sn-phosphatidylcholin (I), gegebenenfalls kombiniert mit
c) synthetischem, zu mehr als 95 % reinen Natrium-1,2-di-(9-cis-octadecenoyl)-3-sn-phosphatidyl-S-serin (II) und
d) für intravenöse Darreichungsformen geeignete, wasserlösliche Hilfsstoffe.

Die Erfindung betrifft in erster Linie pharmazeutische Zusammensetungen in Form von intravenös applizierbaren Liposomendispersionen enthaltend
a) den Zink-Phthalocyaninkomplex,
b) 70 Gew.-% (bezogen auf Komponente c)) synthetisches, zu mehr als 95 % reines 1-n-Hexadecanoyl-2-(9-cis-octadecenoyl)-3-sn-phosphatidylcholin (I), kombiniert mit
c) 30 Gew.-% (bezogen auf Komponente b)) synthetischem, zu mehr als 95 % reinen Natrium-1,2-di-(9-cis-octadecenoyl)-3-sn-phosphatidyl-S-serin (II) und
d) eine pharmazeutisch annehmbare Trägerflüssigkeit und gegebenenfalls für intravenöse Darreichungsformen geeignete, wasserlösliche Hilfsstoffe.

Die Erfindung betrifft insbesondere ebenfalls pharmazeutische Zusammensetzungen in Form von Lyophilisaten enthaltend
a) den Zink-Phthalocyaninkomplex,
b) ca. 70 Gew.-% (bezogen auf die Komponente c)) synthetisches, zu mehr als 95 % reines 1-n-Hexadecanoyl-2-(9-cis-octadecenoyl)-3-sn-phosphatidylcholin (I) kombiniert mit
c) ca. 30 Gew.-% (bezogen auf die Komponente b)) synthetischem, zu mehr als 95 % reinen Natrium-1,2-di-(9-cis-octadecenoyl)-3-sn-phosphatidyl-S-serin (II) sowie
d) für intravenöse Darreichungsformen geeignete, wasserlösliche Hilfsstoffe.

Die folgenden Beispiele illustrieren die Erfindung.

Beispiel 1: In einem Rundkolben werden 2 ml Piperidin, 1 mg Zink-Phthalocyanin, 70 mg mindestens 95 % reines 1-n-Hexadecanoyl-2-(9-cis-octadecenoyl)-3-sn-phosphatidylcholin und 30 mg mindestens 95 % reines 1,2-Di-(9-cis-octadecenoyl)-3-sn-phosphatidylserin gelöst. Man sterilfiltriert diese Lösung über ACRODISC Membranfilter (2,2 x 10⁻⁷ m) und füllt die klare Lösung in Vialen ab.

Nachdem die Lösung bei -40°C eingefroren ist, wird die Viale, bis eine Temperatur von 25°C erreicht ist, im Vakuum getrocknet und unter Argonatmosphäre verschlossen.

Vor dem Gebrauch wird zu diesem Trockenpräparat (Lyophilisat) bei Raumtemperatur mit einer sterilen Spritze 1,5 ml sterile, calcium- und magnesiumfreie, phosphatgepufferte (pH 7,2-7,4) Kochsalzlösung (Dulbecco) gegeben und die Viale auf einem standardisierten Laborschüttler (Vortex, Stufe 7) eine Minute lang geschüttelt. Die entstandene Liposomendispersion ist bei 4°C lagerfähig und eignet sich zur intravenösen Applikation.

Beispiel 2: In einem Rundkolben werden in 2 ml Piperidin je nach Ansatz 0,1 bis 1 mg Zink-Phthalocyanin und 15 bis 400 mg einer Gewichtsmischung sieben zu drei von 95 bis 100 % reinem 1-n-Hexadecanoyl-2-(9-cis-octadecenoyl)-3-sn-phosphatidylcholin und 95 bis 100 % reinem 1,2-Di-(9-cis-octadecenoyl)-3-sn-phosphatidylserin gelöst. Man sterilfiltriert diese Lösung über ACRODISC Membranfilter (2,2 x 10⁻⁷) und füllt die klare Lösung in Vialen ab.

Die Viale wird bei 150 Upm zum Rotieren gebracht und das Lösungsmittel in einem Strom von gereinigtem, bei 1 bar filtriertem Stickstoff abgeblasen.

Anschliessend wird die Viale im Vakuum bei 6,0 x 10⁻² bar evakuiert. Die Viale wird unter Argon-Schutzatmosphäre verschlossen. Vor Gebrauch wird zu diesem hergestellten Film bei Raumtemperatur mit einer Spritze 1,5 ml sterile calcium- und magnesiumfreie, phosphatgepufferte (pH 7,2-7,4) Kochsalzlösung (Dulbecco) gegeben und die Viale auf einem standartisierten Laborschüttler (Stufe 7) zehn Minuten lang geschüttelt. Die entstandene Liposomendispersion ist bei 4°C lagerfähig und eignet sich zur intravenösen Applikation.

Beispiel 3: Analog Beispiel 1 kann man die bei Beispiel 2 erwähnten Lösungen nach Einfrieren im Vakuum trocknen und Lyophilisate herstellen.

Beispiel 4: Analog Beispiel 2 und 3 kann man auch Lösungen herstellen und zwar mit 5:5 bis 9:1 Gewichtsmischungen von 1-n-Hexadecanoyl-2-(9-cis-octadecenoyl)-3-sn-phosphatidylcholin und 1,2-Di-(9-cis-octadecenoyl)-phosphatidylserin.

Beispiel 5: In einem Rundkolben werden in 0,5 ml tert-Butanol, 125 ml 95 % reines 1-n-Hexadecanoyl-2-(9-cis-octadecenoyl)-3-sn-phosphatidylcholin (Avanti, Polar Lipids) gelöst. Separat werden in einem zweiten Rundkolben 0,05 mg Zink-Phthalocyanin in 0,5 ml sterilem Dimethylsulfoxid gelöst.

Man vereinigt beide Lösungen und filtriert diese steril durch Membranfilter ACRODISC (2,2 x 10⁻⁷ m). Nach Abfüllen der klaren Lösung in einer Viale wird diese bei -80°C eingefroren. Die Viale wird, bis eine Temperatur von 25°C erreicht ist, im Vakuum getrocknet und unter Argonatmosphäre verschlossen.

Die weiteren Verfahrensschritte werden analog Beispiel 1 vorgenommen.

Beispiel 6: In einem Rundkolben werden in 0,5 ml tert-Butanol, 12,5 bis 25 mg 95 % reines 1-n-Hexadecanoyl-2-(9-cis-octadecenoyl)-3-sn-phosphtidylcholin (Avanti, Polar Lipids) gelöst. Separat werden in einem zweiten Rundkolben 0,05 mg Zink-Phthalocyanin in 0,5 ml sterilem Dimethylsulfoxid gelöst.

Analog Beispiel 5 werden beide Lösungen vereinigt, über ACRODISC sterilfiltriert und in Vialen abgefüllt. Die Viale wird bei 150 Upm zum Rotieren gebracht und das Lösungsmittel in einem Strom von gereinigtem, bei 1 bar filtriertem Stickstoff abgeblasen. Anschliessend wird die Viale im Vakuum bei 6,0 x 10⁻² mbar evakuiert. Die Viale wird unter Argon-Schutzgasatmosphäre verschlossen.

Die weiteren Verfahrensschritte erfolgen analog Beispiel 2.

Beispiel 7: In einem Rundkolben werden 1 ml steriles Piperidin, 0,5 mg Zink-Phthalocyanin und 250 mg 95 % reines 1-n-Hexadecanoyl-2-(9-cis-octadecenoyl)-3-sn-phosphatidylcholin gelöst. Man sterilfiltriert diese Lösung über ACRODISC Membranfilter (2,2 x 10⁻⁷ m) und füllt die klare Lösung in Vialen ab.

Analog Beispiel 5 kann man ein Lyophilisat oder wahlweise analog Beispiel 6 einen Filmrückstand herstellen, welchen man anschliessend analog Beispiel 1 oder 2 in eine intravenös applizierbare Liposomendispersion überführt.

Beispiel 8: Analog Beispiel 7 lässt sich ein Lyophilisat oder ein Filmrückstand durch Auflösen von 3 mg Zink-Phthalocyanin in 1 ml sterilem Piperidin herstellen. Aus diesen Trockenpräparaten wird anschliessend analog Beispiel 1 oder 2 eine intravenös applizierbare Liposomendispersion hergestellt.

Beispiel 9: In einem Masskolben werden bei Raumtemperatur in 400 ml Piperidin puriss. je nach Ansatz 200-400 mg Zink Phthalocyanin gelöst und auf 500 ml aufgefüllt. In einem zweiten Kolben werden bei Raumtemperatur je nach Ansatz 120-240 mg einer Gewichtsmischung 7:3 von 95-100 % reinem 1-n-Hexadecanoyl-2-(9-cis-octadecenoyl)-3-sn-phosphatidylcholin und 95-100 % reinem 1,2-Di-(9-cis-octadecenoyl)-3-sn-phosphatidyl-S-serin in 6 ml Piperidin puriss. gelöst und mit 6-12 ml der obigen Zink-Phthalocyanin Lösung versetzt. Man sterilfiltriert diese Lösung über ACRODISC Membranfilter (2,2 x 10⁻⁷) und füllt die klare Lösung in Vialen ab.

Die weiteren Verfahrensschritte erfolgen analog Beispiel 1.

Beispiel 10: In einem Masskolben werden bei Raumtemperatur in 400 ml Piperidin puriss. je nach Ansatz 200-400 mg Zink-Phthalocyanin gelöst und auf 500 ml aufgefüllt. In einem zweiten Kolben werden je nach Ansatz 120-240 mg einer Gewichtsmischung 7:3 von 95-100 % reinem 1-n-Hexadecanoyl-2-(9-cis-octadecenoyl)-3-sn-phosphatidylcholin und 95-100 % reinem 1,2-Di-(9-cis-octadecenoyl)-3-sn-phosphatidyl-S-serin in 6 ml eines Piperidin/Wasser-Gemisches (2-10 %) bei 0-4°C gelöst und bei dieser Temperatur 6-12 ml der obigen Zink-Phthalocyanin Lösung zugetropft. Man sterilfiltriert diese Lösung über ACRODISC Membranfilter (2,2 x 10⁻⁷) und füllt die klare Lösung in Vialen ab.

Die weiteren Verfahrensschritte erfolgen analog Beispiel 2.

Beispiel 11: Eine nach Beispiel 9 oder Beispiel 10 hergestellte Lösung von Zink-Phthalocyanin und Lipidgemisch wird nach der Sterilfiltration nicht in Vialen abgefüllt, sondern in einem Batchverfahren lyophilisiert. Aliquote Mengen dieses Lyophilisats werden analog Beispiel 1 in eine Liposomendispersion überführt.

Beispiel 12: In einem Rundkolben werden in 205 ml frisch destilliertem Piperidin, 100 mg Zink-Phthalocyanin, 7000 mg 95 % reines 1-n-Hexadecanoyl-2-(9-cis-octadecanoyl)-3-sn-phosphatidyl-cholin (POPC) und 3000 mg 95 % reines Natrium-1,2-di(9-cis-octadecanoyl)-3-sn-phosphatidyl-S-serin (OOPS) gelöst.

Dieser Lösung wird unter Rühren 2000 ml 9.75 % G/V wässrige Lactoselösung zugefügt. Nachdem der pH-Wert mittels Zugabe von verdünnter HCl auf 7.0 eingestellt ist, wird die entstandene Dispersion mit Hilfe eines Millipore Minitan® tangentiellen Dialysegeräts bis zu einer Konzentration von 0.5 mg Zn-Phthalocyanin/ml konzentriert und gegen 2000 ml 9.75 % Lactose dialysiert.

Die entstandene lactosehaltige Dispersion wird dann mittels ACRODISC Membranfilter (2,2 x 10-7) sterilfiltriert und in Vials (2 ml pro Vial enthaltend 1 mg Zn-Phthalocyanin) abgefüllt. Nachdem die Dispersion bei -40°C eingefroren ist, werden die Vials, bis eine Temperatur von 25°C erreicht ist, im Vakuum getrocknet und unter Argonatmosphäre verschlossen.

Die entstandenen Trockenpräparate sind mehrere Jahre bei 4°C lagerfähig.

Vor der Applikation wird in ein Trockenpräparat (Lyophilisat) in einem Vial bei Raumtemperatur mit einer sterilen Spritze 2,0 ml Wasser injiziert. Nach einer maximalen Auflösungszeit von einer Minute entsteht eine Liposomen-Dispersion, die sich zur intravenösen Applikation eignet.

Beispiel 13: Analog Beispiel 12, kann man das Verfahren mit 10000 mg POPC durchführen.

Beispiel 14: Analog Beispiel 13, kann man das Verfahren mit 300 mg POPC oder 210 mg POPC und 90 mg OOPS durchführen.

Beispiel 15: In einem Kolben werden 35 g POPC und 15 g OOPS in 500 ml tertiär-Butanol unter Rühren bei 40°C gelöst. In einem weiteren Kolben werden 0.5 g Zn-Pththalocyanin in 125 ml NMP gelöst (Ultraschallbad).

Beide Lösungen werden gemischt, wobei die Farbstofflösung in die tertiär-Butanollösung eingegossen wird. Die Mischung wird auf 40°C erwärmt, um eine klare Lösung zu erhalten. Diese Lösung wird mit Hilfe eines dynamischen Mischers mit 10 Liter Lactose-Medium, welche auf 4°C gekühlt ist und pro Liter 94.9 g Lactose zur Injektion und 24 g NaCl enthält, gemischt.

Die organische Phase wird mit einer Geschwindigkeit von 107 ml/min, die Lactoselösung mit 1714 ml/min in den dynamischen Mischer gepumpt, welcher mit 3 bar Druck angetrieben wird.

Die entstandene blaue, leicht opaleszente Dispersion (10625 ml) wird mit Hilfe eines Millipore tangentiellen Dialysegeräts bis 1 Liter konzentriert und dann gegen 10 l Lactose-Medium dialysiert.

Das weitere Vorgehen ist in Beispiel 12 beschrieben.

Beispiel 16: Analog Beispiel 15 werden 3.5 g POPC und 1.5 g OOPS in 100 ml t-Butanol gelöst und mit 25 ml NMP und 100 mg Zink-Phthalocyanin gemischt. Die organische Phase wird dann mit 2 l Lactose-Medium (25 g Lactose und 0.064 g NaCl pro Liter) gemischt. Das Dialysemedium hat die gleiche Zusammensetzung.

Beispiel 17: Analog Beispiel 16, wird die organische Phase mit 2 l Lactose-Medium enthaltend 50 g Lactose und 0.127 g NaCl pro Liter gemischt.

Beispiel 18: Analog Beispiel 15 werden 10.5 g POPC und 4.5 g OOPS in 200 ml t-Butanol gelöst und mit 50 ml NMP mit 200 mg Zink-Phthalocyanin gemischt. Die organische Phase wird mit 4 l Lactose-Medium enthaltend 37.5 g Lactose und 0.095 g NaCl pro Liter gemischt. Das Dialysemedium hat die gleiche Zusammensetzung.

Beispiel 19: Analoge Beispiel 18 wird die organische Phase mit 4 l Lactose Lösung enthaltende 75 g Lactose und 0.1905 g NaCl pro Liter gemischt. Das Dialysemedium hat die gleiche Zusammensetzung.

Beispiel 20: Analog Beispiel 15, werden 3.5 g POPC und 1.5 g OOPS in 200 ml t-Butanol gelöst und mit 50 ml NMP mit 200 mg Zinkphthalocyanin gemischt. Die organische Phase wird mit 4 l Lactose Medium enthaltend 25 g Lactose und 0.064 g NaCl pro Liter gemischt. Das Dialysemedium hat die gleiche Zusammensetzung.

## Patentansprüche (Patentansprüche für folgende(n) Vertragsstaat(en): AT, BE, CH, DE, DK, FR, GB, IT, LI, LU, NL, SE)

1. Pharmazeutische Zusammensetzungen in Form von parenteral applizierbaren Liposomendispersionen enthaltend
a) den Zink-Phthalocyaninkomplex,
b) ein synthetisches, zu mehr als 90 % reines Phospholipid der Formel 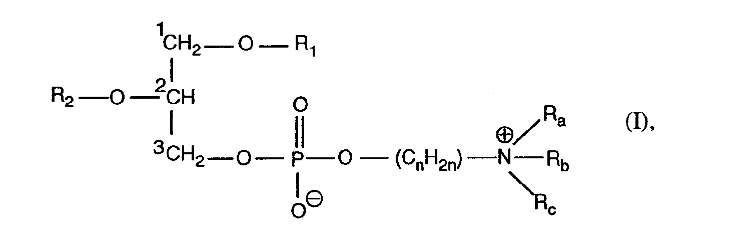 worin R₁ C₁₀-C₂₀-Alkanoyl mit gerader Anzahl an C-Atomen, R₂ C₁₀-C₂₀-Alkenoyl mit gerader Anzahl an C-Atomen, Rₐ, R_{b} und R_{c} Wasserstoff oder C₁-C₄-Alkyl und n eine ganze Zahl von zwei bis vier darstellen, gegebenenfalls kombiniert mit einem
c) synthetischen, zu mehr als 90 % reinen Phospholipid der Formel 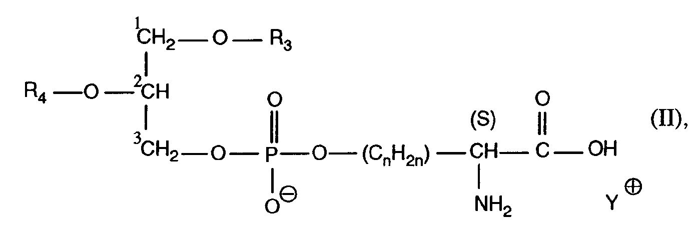 worin R₃ und R₄ unabhängig voneinander C₁₀-C₂₀-Alkenoyl mit gerader Anzahl an C-Atomen, n eine ganze Zahl von eins bis drei und Y^{⊕} das Kation einer pharmazeutisch annehmbaren Base darstellen, und
d) eine pharmazeutisch annehmbare Trägerflüssigkeit und gegebenenfalls für parenterale Darreichungsformen geeignete wasserlösliche Hilfsstoffe.

2. Pharmazeutische Zusammensetzungen gemäss Anspruch 1 in Form von intravenös applizierbaren Liposomendispersionen enthaltend
a) den Zink-Phthalocyaninkomplex,
b) ein synthetisches, zu mehr als 95 % reines Phospholipid der Formel I, worin R₁ n-Dodecanoyl, n-Tetradecanoyl, n-Hexadecanoyl oder n-Octadecanoyl und R₂ 9-cis-Dodecenoyl, 9-cis-Tetradecenoyl, 9-cis-Hexadecenoyl, 9-cis-Octadecenoyl oder 9-cis-Icosenoyl, Rₐ, R_{b} und R_{c} Methyl und n zwei bedeuten, gegebenenfalls kombiniert mit einem
c) synthetischen, zu mehr als 95 % reinen Phospholipid der Formel II, worin R₃ und R₄ identische Bedeutungen haben und 9-cis-Dodecenoyl, 9-cis-Tetradecenoyl, 9-cis-Hexadecenoyl, 9-cis-Octadecenoyl oder 9-cis-Icosenoyl bedeuten, n eins und Y^{⊕} das Natriumion ist, und
d) eine pharmazeutisch annehmbare Trägerflüssigkeit und gegebenenfalls für intravenöse Darreichungsformen geeignete wasserlösliche Hilfsstoffe.

3. Pharmazeutische Zusammensetzungen gemäss Anspruch 1 in Form von intravenös applizierbaren Liposomendispersionen enthaltend
a) den Zink-Phthalocyaninkomplex,
b) synthetisches, zu mehr als 95 % reines 1-n-Hexadecanoyl-2-(9-cis-octadecenoyl)-3-sn-phosphatidylcholin (I), gegebenenfalls kombiniert mit
c) synthetischem, zu mehr als 95 % reinen Natrium-1,2-di-(9-cis-octadecenoyl)-3-sn-phosphatidyl-S-serin (II), und
d) eine pharmazeutisch annehmbare Trägerflüssigkeit und gegebenenfalls für intravenöse Darreichungsformen geeignete wasserlösliche Hilfsstoffe.

4. Pharmazeutische Zusammensetzungen gemäss Anspruch 1 in Form von intravenös applizierbaren Liposomendispersionen enthaltend
a) den Zink-Phthalocyaninkomplex,
b) 70 Gew.-% (bezogen auf Komponente c)) synthetisches, zu mehr als 95 % reines 1-n-Hexadecanoyl-2-(9-cis-octadecenoyl)-3-sn-phosphatidylcholin (I), kombiniert mit
c) 30 Gew.-% (bezogen auf Komponente b)) synthetischem, zu mehr als 95 % reinen Natrium-1,2-di-(9-cis-octadecenoyl)-3-sn-phosphatidyl-S-serin (II) und
d) eine pharmazeutisch annehmbare Trägerflüssigkeit und gegebenenfalls für intravenöse Darreichungsformen geeignete, wasserlösliche Hilfsstoffe.

5. Pharmazeutische Zusammensetzungen in Form von Trockenpräparaten enthaltend
a) den Zink-Phthalocyaninkomplex,
b) ein synthetisches, zu mehr als 90 % reines Phospholipid der Formel I, worin R₁, R₂, Rₐ, R_{b} und R_{c} und n die in Anspruch 1 genannten Bedeutungen haben, gegebenenfalls kombiniert mit einem
c) synthetischen, zu mehr als 90 % reinen Phospholipid der Formel II, worin R₃ und R₄, Y^{⊕} und n die in Anspruch 1 genannten Bedeutungen haben, und gegebenenfalls
d) für intravenöse Darreichungsformen geeignete, wasserlösliche Hilfsstoffe.

6. Pharmazeutische Zusammensetzungen gemäss Anspruch 5 in Form von Lyophilisaten enthaltend
a) den Zink-Phthalocyaninkomplex,
b) 70 Gew.-% (bezogen auf die Komponente c)) synthetisches, zu mehr als 95 % reines 1-n-Hexadecanoyl-2-(9-cis-octadecenoyl)-3-sn-phosphatidylchol in (I) kombiniert mit
c) 30 Gew.-% (bezogen auf die Komponente b)) synthetischem, zu mehr als 95 % reinen Natrium-1,2-di-(9-cis-octadecenoyl)-3-sn-phosphatidyl-S-serin (II) sowie
d) für intravenöse Darreichungsformen geeignete, wasserlösliche Hilfsstoffe.

7. Verfahren zur Herstellung einer pharmazeutischen Zusammensetzung in Form einer parenteral applizierbaren Liposomendispersion, dadurch gekennzeichnet, dass man ein Trockenpräparat enthaltend
a) den Zink-Phthalocyaninkomplex,
b) ein synthetisches, zu mehr als 90 % reines Phospholipid der Formel I, worin R₁, R₂, Rₐ, R_{b} und R_{c} und n die genannten Bedeutungen haben, gegebenenfalls kombiniert mit einem
c) synthetischen, zu mehr als 90 % reinen Phospholipid der Formel II, worin R₃, R₄, Y^{⊕} und n die genannten Bedeutungen haben, und gegebenenfalls
d) für parenterale Darreichungsformen geeignete, wasserlösliche Hilfsstoffe in wässriger Phase dispergiert und gegebenenfalls die erhältliche wässrige Dispersion auf pH 7,0-7,8 abpuffert und/oder eine Fraktion von Liposomen mit einem gewünschten Bereich des Durchmessers isoliert.

8. Verfahren zur Herstellung einer pharmazeutischen Zusammensetzung in Form eines Trockenpräparates, dadurch gekennzeichnet, dass man
a) den Zink-Phthalocyaninkomplex und
b) ein synthetisches, zu mehr als 90 % reines Phospholipid der Formel I, worin R₁, R₂, Rₐ, R_{b} und R_{c} und n die genannten Bedeutungen haben, gegebenenfalls kombiniert mit einem
c) synthetischen, zu mehr als 90 % reinen Phospholipid der Formel II, worin R₃ und R₄, Y^{⊕} und n die genannten Bedeutungen haben, in einem pharmazeutisch annehmbaren organischen Lösungsmittel löst, dessen Restmenge in einem Trockenpräparat toxikologisch unbedenklich ist und gegebenenfalls
d) Trägerflüssigkeit und für parenterale Darreichungsformen geeignete wasserlösliche Hilfsstoffe zusetzt und das Lösungsmittel oder Lösungsmittelgemisch entfernt.

9. Verfahren gemäss Anspruch 8, dadurch gekennzeichnet, dass man a) den Zink-Phthalocyaninkomplex, b) das Phospholipid der Formel I und gegebenenfalls c) das Phospholipid der Formel II in Piperidin löst.

10. Verwendung von pharmazeutischen Zusammensetzungen in Form von Trockenpräparaten gemäss Anspruch 5 zur Herstellung einer parenteral applizierbaren Liposomendispersion.

11. Verwendung des Zink-Phthalocyaninkomlexes, des Phospholipids der Formel I und gegebenenfalls der Formel II zur Herstellung von Trockenpräparaten.

12. Pharmazeutische Zusammensetzungen gemäss Anspruch 1 zur Anwendung in einem Verfahren zur therapeutischen Behandlung des menschlichen oder tierischen Körpers.

## Patentansprüche (Patentansprüche für folgende(n) Vertragsstaat(en): ES, GR)

1. Verfahren zur Herstellung von pharmazeutischen Zusammensetzungen in Form von parenteral applizierbaren Liposomendispersionen enthaltend
a) den Zink-Phthalocyaninkomplex,
b) ein synthetisches, zu mehr als 90 % reines Phospholipid der Formel 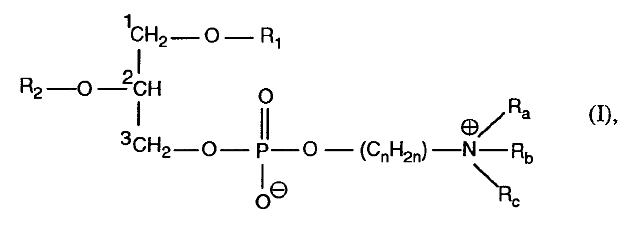 worin R₁ C₁₀-C₂₀-Alkanoyl mit gerader Anzahl an C-Atomen, R₂ C₁₀-C₂₀-Alkenoyl mit gerader Anzahl an C-Atomen, Rₐ, R_{b} und R_{c} Wasserstoff oder C₁-C₄-Alkyl und n eine ganze Zahl von zwei bis vier darstellen, gegebenenfalls kombiniert mit einem
c) synthetischen, zu mehr als 90 % reinen Phospholipid der Formel 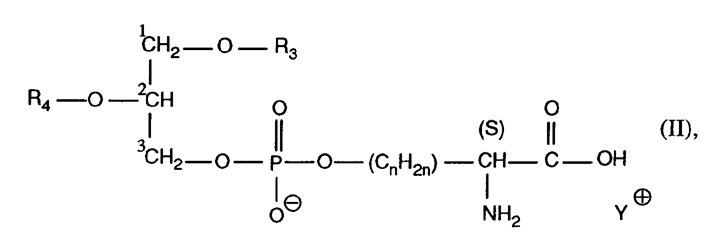 worin R₃ und R₄ unabhängig voneinander C₁₀-C₂₀-Alkenoyl mit gerader Anzahl an C-Atomen, n eine ganze Zahl von eins bis drei und Y^{⊕} das Kation einer pharmazeutisch annehmbaren Base darstellen, und
d) eine pharmazeutisch annehmbare Trägerflüssigkeit und gegebenenfalls für parenterale arreichungsformen geeignete wasserlösliche Hilfsstoffe, dadurch gekennzeichnet, dass man ein Trockenpräparat enthaltend
a) den Zink-Phthalocyaninkomplex,
b) ein synthetisches, zu mehr als 90 % reines Phospholipid der Formel I, worin R₁, R₂, Rₐ, R_{b} und R_{c} und n die genannten Bedeutungen haben, gegebenenfalls kombiniert mit einem
c) synthetischen, zu mehr als 90 % reinen Phospholipid der Formel II, worin R₃, R₄, Y^{⊕} und n die genannten Bedeutungen haben, und gegebenenfalls
d) für parenterale Darreichungsformen geeignete, wasserlösliche Hilfsstoffe in wässriger Phase dispergiert und gegebenenfalls die erhältliche wässrige Dispersion auf pH 7,0-7,8 abpuffert und/oder eine Fraktion von Liposomen mit einem gewünschten Bereich des Durchmessers isoliert.

2. Verfahren zur Herstellung pharmazeutischen Zusammensetzungen gemäss Anspruch 1 in Form von intravenös applizierbaren Liposomendispersionen enthaltend
a) den Zink-Phthalocyaninkomplex,
b) ein synthetisches, zu mehr als 95 % reines Phospholipid der Formel I, worin R₁ n-Dodecanoyl, n-Tetradecanoyl, n-Hexadecanoyl oder n-Octadecanoyl und R₂ 9-cis-Dodecenoyl, 9-cis-Tetradecenoyl, 9-cis-Hexadecenoyl, 9-cis-Octadecenoyl oder 9-cis-Icosenoyl, Rₐ, R_{b} und R_{c} Methyl und n zwei bedeuten, gegebenenfalls kombiniert mit einem
c) synthetischen, zu mehr als 95 % reinen Phospholipid der Formel II, worin R₃ und R₄ identische Bedeutungen haben und 9-cis-Dodecenoyl, 9-cis-Tetradecenoyl, 9-cis-Hexadecenoyl, 9-cis-Octadecenoyl oder 9-cis-Icosenoyl bedeuten, n eins und Y^{⊕} das Natriumion ist, und
d) eine pharmazeutisch annehmbare Trägerflüssigkeit und gegebenenfalls für intravenöse Darreichungsformen geeignete wasserlösliche Hilfsstoffe, dadurch gekennzeichnet, dass man die im Anspruch 1 bezeichneten Massnahmen durchführt.

3. Verfahren zur Herstellung von pharmazeutischen Zusammensetzungen gemäss Anspruch 1 in Form von intravenös applizierbaren Liposomendispersionen enthaltend
a) den Zink-Phthalocyaninkomplex,
b) synthetisches, zu mehr als 95 % reines 1-n-Hexadecanoyl-2-(9-cis-octadecenoyl)-3-sn-phosphatidylcholin (I), gegebenenfalls kombiniert mit
c) synthetischem, zu mehr als 95 % reinen Natrium-1,2-di-(9-cis-octadecenoyl)-3-sn-phosphatidyl-S-serin (II), und
d) eine pharmazeutisch annehmbare Trägerflüssigkeit und gegebenenfalls für intravenöse Darreichungsformen geeignete wasserlösliche Hilfsstoffe, dadurch gekennzeichnet, dass man die im Anspruch 1 bezeichneten Massnahmen durchführt.

4. Verfahren zur Herstellung von pharmazeutischen Zusammensetzungen gemäss Anspruch 1 in Form von intravenös applizierbaren Liposomendispersionen enthaltend
a) den Zink-Phthalocyaninkomplex,
b) 70 Gew.-% (bezogen auf Komponente c)) synthetisches, zu mehr als 95 % reines 1-n-Hexadecanoyl-2-(9-cis-octadecenoyl)-3-sn-phosphatidylcholin (I), kombiniert mit
c) 30 Gew.-% (bezogen auf Komponente b)) synthetischem, zu mehr als 95 % reinen Natrium-1,2-di-(9cis-octadecenoyl)-3-sn-phosphatidyl-S-serin (II) und
d) eine pharmazeutisch annehmbare Trägerflüssigkeit und gegebenenfalls für intravenöse Darreichungsformen geeignete, wasserlösliche Hilfsstoffe, dadurch gekennzeichnet, dass man die im Anspruch 1 bezeichneten Massnahmen durchführt.

5. Verfahren zur Herstellung von pharmazeutischen Zusammensetzungen in Form von Trockenpräparaten enthaltend
a) den Zink-Phthalocyaninkomplex,
b) ein synthetisches, zu mehr als 90 % reines Phospholipid der Formel I, worin R₁, R₂, Rₐ, R_{b} und R_{c} und n die in Anspruch 1 genannten Bedeutungen haben, gegebenenfalls kombiniert mit einem
c) synthetischen, zu mehr als 90 % reinen Phospholipid der Formel II, worin R₃ und R₄, Y^{⊕} und n die in Anspruch 1 genannten Bedeutungen haben, und gegebenenfalls
d) für intravenöse Darreichungsformen geeignete, wasserlösliche Hilfsstoffe, dadurch gekennzeichnet, dass man die im Anspruch 1 bezeichneten Massnahmen durchführt.

6. Verfahren zur Herstellung von pharmazeutischen Zusammensetzungen gemäss Anspruch 5 in Form von Lyophilisaten enthaltend
a) den Zink-Phthalocyaninkomplex,
b) 70 Gew.-% (bezogen auf die Komponente c)) synthetisches, zu mehr als 95 % reines 1-n-Hexadecanoyl-2-(9-cis-octadecenoyl)-3-sn-phosphatidylchol in (I) kombiniert mit
c) 30 Gew.-% (bezogen auf die Komponente b)) synthetischem, zu mehr als 95 % reinen Natrium-1,2-di-(9-cis-octadecenoyl)-3-sn-phosphatidyl-S-serin (II) sowie
d) für intravenöse Darreichungsformen geeignete, wasserlösliche Hilfsstoffe, dadurch gekennzeichnet, dass man die im Anspruch 1 bezeichneten Massnahmen durchführt.

7. Verfahren zur Herstellung einer pharmazeutischen Zusammensetzung in Form eines Trockenpräparates, dadurch gekennzeichnet, dass man
a) den Zink-Phthalocyaninkomplex und
b) ein synthetisches, zu mehr als 90 % reines Phospholipid der Formel I, worin R₁, R₂, Rₐ, R_{b} und R_{c} und n die genannten Bedeutungen haben, gegebenenfalls kombiniert mit einem
c) synthetischen, zu mehr als 90 % reinen Phospholipid der Formel II, worin R₃ und R₄, Y^{⊕} und n die genannten Bedeutungen haben, in einem pharmazeutisch annehmbaren organischen Lösungsmittel löst, dessen Restmenge in einem Trockenpräparat toxikologisch unbedenklich ist und gegebenenfalls
d) Trägerflüssigkeit und für parenterale Darreichungsformen geeignete wasserlösliche Hilfsstoffe zusetzt und das Lösungsmittel oder Lösungsmittelgemisch entfernt.

8. Verfahren gemäss Anspruch 8, dadurch gekennzeichnet, dass man a) den Zink-Phthalocyaninkomplex, b) das Phospholipid der Formel I und gegebenenfalls c) das Phospholipid der Formel II in Piperidin löst.

## Claims (Claims for the following Contracting State(s): AT, BE, CH, DE, DK, FR, GB, IT, LI, LU, NL, SE)

1. A pharmaceutical composition in the form of a parenterally administrable liposome dispersion comprising
a) the zinc-phthalocyanine complex,
b) a synthetic, more than 90 % pure phospholipid of formula 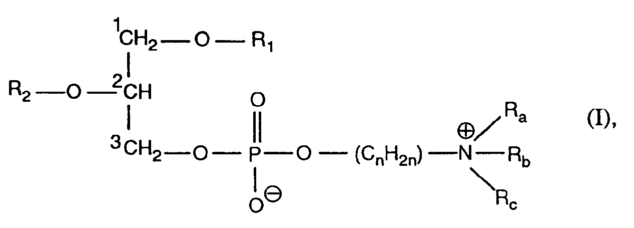 wherein R₁ is C₁₀-C₂₀alkanoyl having an even number of carbon atoms, R₂ is C₁₀-C₂₀alkenoyl having an even number of carbon atoms, Rₐ, R_{b} and R_{c} are hydrogen or C₁-C₄alkyl and n is an integer from two to four, optionally combined with a
c) synthetic, more than 90 % pure phospholipid of formula 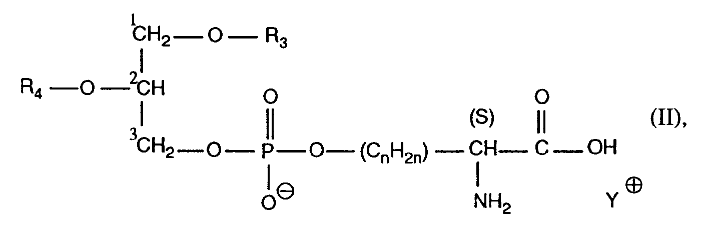 wherein R₃ and R₄ are each independently of the other C₁₀-C₂₀alkenoyl having an even number of carbon atoms, n is an integer from one to three and Y^{⊕} is the cation of a pharmaceutically acceptable base, and
d) a pharmaceutically acceptable carrier liquid and, optionally, water-soluble excipients suitable for parenteral dosage forms.

2. A pharmaceutical composition according to claim 1 in the form of an intravenously administrable liposome dispersion comprising
a) the zinc-phthalocyanine complex,
b) a synthetic, more than 95 % pure phospholipid of formula I, wherein R₁ is n-dodecanoyl, n-tetradecanoyl, n-hexadecanoyl or n-octadecanoyl and R₂ is 9-cis-dodecenoyl, 9-cis-tetradecenoyl, 9-cis-hexadecenoyl, 9-cis-octadecenoyl or 9-cis-icosenoyl, Rₐ, R_{b} and R_{c} are methyl and n is two, optionally combined with a
c) synthetic, more than 95 % pure phospholipid of formula II, wherein R₃ and R₄ are identical and are 9-cis-dodecenoyl, 9-cis-tetradecenoyl, 9-cis-hexadecenoyl, 9-cis-octadecenoyl or 9-cis-icosenoyl, n is one and Y^{⊕} is the sodium ion, and
d) a pharmaceutically acceptable carrier liquid and, optionally, water-soluble excipients suitable for intravenous dosage forms.

3. A pharmaceutical composition according to claim 1 in the form of an intravenously administrable liposome dispersion comprising
a) the zinc-phthalocyanine complex,
b) synthetic, more than 95 % pure 1-n-hexadecanoyl-2-(9-cis-octadecenoyl)-3-sn-phosphatidyl choline (I), optionally combined with
c) synthetic, more than 95 % pure sodium 1,2-di(9-cis-octadecenoyl)-3-sn-phosphatidyl S-serine (II), and
d) a pharmaceutically acceptable carrier liquid and, optionally, water-soluble excipients suitable for intravenous dosage forms.

4. A pharmaceutical composition according to claim 1 in the form of an intravenously administrable liposome dispersion comprising
a) the zinc-phthalocyanine complex,
b) 70 % by weight (based on component c)) synthetic, more than 95 % pure 1-n-hexadecanoyl-2-(9-cis-octadecenoyl)-3-sn-phosphatidyl choline (I), combined with
c) 30 % by weight (based on component b)) synthetic, more than 95 % pure sodium 1,2-di-(9-cis-octadecenoyl)-3-sn-phosphatidyl S-serine (II), and
d) a pharmaceutically acceptable carrier liquid and, optionally, water-soluble excipients suitable for intravenous dosage forms.

5. A pharmaceutical composition in the form of a dry preparation comprising
a) the zinc-phthalocyanine complex,
b) a synthetic, more than 90 % pure phospholipid of formula I, wherein R₁, R₂, Rₐ, R_{b} and R_{c} and also n are as defined in claim 1, optionally combined with a
c) synthetic, more than 90 % pure phospholipid of formula II, wherein R₃, R₄, Y^{⊕} and n are as defined in claim 1, and optionally
d) water-soluble excipients suitable for intravenous dosage forms.

6. A pharmaceutical composition according to claim 5 in the form of a lyophilisate comprising
a) the zinc-phthalocyanine complex,
b) 70 % by weight (based on component c)) synthetic, more than 95 % pure 1-n-hexadecanoyl-2-(9-cis-octadecenoyl)-3-sn-phosphatidyl choline (I), combined with
c) 30 % by weight (based on component b)) synthetic, more than 95 % pure sodium 1,2-di-(9-cis-octadecenoyl)-3-sn-phosphatidyl S-serine (II), and
d) water-soluble excipients suitable for intravenous dosage forms.

7. A process for the preparation of a pharmaceutical composition in the form of a parenterally administrable liposome dispersion, which process comprises dispersing in aqueous phase a dry preparation comprising
a) the zinc-phthalocyanine complex,
b) a synthetic, more than 90 % pure phospholipid of formula I, wherein R₁, R₂, Rₐ, R_{b} and R_{c} and also n are as defined, optionally combined with a
c) synthetic, more than 90 % pure phospholipid of formula II, wherein R₃, R₄, Y^{⊕} and n are as defined, and optionally
d) water-soluble excipients suitable for parenteral dosage forms, and optionally buffering the obtainable aqueous dispersion to pH 7.0-7.8 and/or isolating a liposome fraction having a desired diameter range.

8. A process for the preparation of a pharmaceutical composition in the form of a dry preparation, which process comprises dissolving
a) the zinc-phthalocyanine complex and
b) a synthetic, more than 90 % pure phospholipid of formula I, wherein R₁, R₂, Rₐ, R_{b} and R_{c} and also n are as defined, optionally combined with a
c) synthetic, more than 90 % pure phospholipid of formula II, wherein R₃, R₄, Y^{⊕} and n are as defined, in a pharmaceutically acceptable organic solvent, the residual amount of which in a dry preparation is toxicologically harmless, and optionally
d) adding carrier liquid and water-soluble excipients suitable for parenteral dosage forms, and removing the solvent or solvent mixture.

9. A process according to claim 8, wherein a) the zinc-phthalocyanine complex, b) the phospholipid of formula I and optionally c) the phospholipid of formula II are dissolved in piperidine.

10. The use of a pharmaceutical composition in the form of a dry preparation according to claim 5 for the preparation of a parenterally administrable liposome dispersion.

11. The use of the zinc-phthalocyanine complex, the phospholipid of formula I and optionally formula II for the preparation of a dry preparation.

12. A pharmaceutical composition according to claim 1 for use in a method for the therapeutic treatment of the human or animal body.

## Claims (Claims for the following Contracting State(s): ES, GR)

1. A process for the preparation of a pharmaceutical composition in the form of a parenterally administrable liposome dispersion comprising
a) the zinc-phthalocyanine complex,
b) a synthetic, more than 90 % pure phospholipid of formula 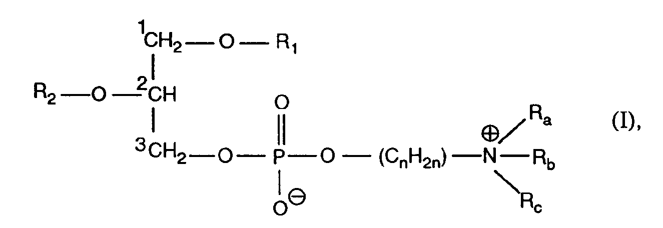 wherein R₁ is C₁₀-C₂₀alkanoyl having an even number of carbon atoms, R₂ is C₁₀-C₂₀alkenoyl having an even number of carbon atoms, Rₐ, R_{b} and R_{c} are hydrogen or C₁-C₄alkyl and n is an integer from two to four, optionally combined with a
c) synthetic, more than 90 % pure phospholipid of formula 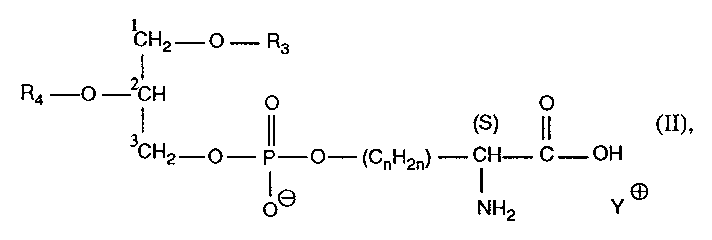 wherein R₃ and R₄ are each independently of the other C₁₀-C₂₀alkenoyl having an even number of carbon atoms, n is an integer from one to three and Y^{⊕} is the cation of a pharmaceutically acceptable base, and
d) a pharmaceutically acceptable carrier liquid and, optionally, water-soluble excipients suitable for parenteral dosage forms,
which process comprises dispersing in aqueous phase a dry preparation comprising
a) the zinc-phthalocyanine complex,
b) a synthetic, more than 90 % pure phospholipid of formula I, wherein R₁, R₂, Rₐ, R_{b} and R_{c} and also n are as defined, optionally combined with a
c) synthetic, more than 90 % pure phospholipid of formula II, wherein R₃, R₄, Y^{⊕} and n are as defined, and optionally
d) water-soluble excipients suitable for parenteral dosage forms, and optionally buffering the obtainable aqueous dispersion to pH 7.0-7.8 and/or isolating a liposome fraction having a desired diameter range.

2. A process for the preparation of a pharmaceutical composition according to claim 1 in the form of an intravenously administrable liposome dispersion comprising
a) the zinc-phthalocyanine complex,
b) a synthetic, more than 95 % pure phospholipid of formula I, wherein R₁ is n-dodecanoyl, n-tetradecanoyl, n-hexadecanoyl or n-octadecanoyl and R₂ is 9-cis-dodecenoyl, 9-cis-tetradecenoyl, 9-cis-hexadecenoyl, 9-cis-octadecenoyl or 9-cis-icosenoyl, Rₐ, R_{b} and R_{c} are methyl and n is two, optionally combined with a
c) synthetic, more than 95 % pure phospholipid of formula II, wherein R₃ and R₄ are identical and are 9-cis-dodecenoyl, 9-cis-tetradecenoyl, 9-cis-hexadecenoyl, 9-cis-octadecenoyl or 9-cis-icosenoyl, n is one and Y^{⊕} is the sodium ion, and
d) a pharmaceutically acceptable carrier liquid and, optionally, water-soluble excipients suitable for intravenous dosage forms,
wherein the steps indicated in claim 1 are carried out.

3. A process for the preparation of a pharmaceutical composition according to claim 1 in the form of an intravenously administrable liposome dispersion comprising
a) the zinc-phthalocyanine complex,
b) synthetic, more than 95 % pure 1-n-hexadecanoyl-2-(9-cis-octadecenoyl)-3-sn-phosphatidyl choline (I), optionally combined with
c) synthetic, more than 95 % pure sodium 1,2-di(9-cis-octadecenoyl)-3-sn-phosphatidyl S-serine (II), and
d) a pharmaceutically acceptable carrier liquid and, optionally, water-soluble excipients suitable for intravenous dosage forms,
wherein the steps indicated in claim 1 are carried out.

4. A process for the preparation of a pharmaceutical composition according to claim 1 in the form of an intravenously administrable liposome dispersion comprising
a) the zinc-phthalocyanine complex,
b) 70 % by weight (based on component c)) synthetic, more than 95 % pure 1-n-hexadecanoyl-2-(9-cis-octadecenoyl)-3-sn-phosphatidyl choline (I), combined with
c) 30 % by weight (based on component b)) synthetic, more than 95 % pure sodium 1,2-di-(9-cis-octadecenoyl)-3-sn-phosphatidyl S-serine (II), and
d) a pharmaceutically acceptable carrier liquid and, optionally, water-soluble excipients suitable for intravenous dosage forms,
wherein the steps indicated in claim 1 are carried out.

5. A process for the preparation of a pharmaceutical composition in the form of a dry preparation comprising
a) the zinc-phthalocyanine complex,
b) a synthetic, more than 90 % pure phospholipid of formula I, wherein R₁, R₂, Rₐ, R_{b} and R_{c} and also n are as defined in claim 1, optionally combined with a
c) synthetic, more than 90 % pure phospholipid of formula II, wherein R₃, R₄, Y^{⊕} and n are as defined in claim 1, and optionally
d) water-soluble excipients suitable for intravenous dosage forms,
wherein the steps indicated in claim 1 are carried out.

6. A process for the preparation of a pharmaceutical composition according to claim 5 in the form of a lyophilisate comprising
a) the zinc-phthalocyanine complex,
b) 70 % by weight (based on component c)) synthetic, more than 95 % pure 1-n-hexadecanoyl-2-(9-cis-octadecenoyl)-3-sn-phosphatidyl choline (I), combined with
c) 30 % by weight (based on component b)) synthetic, more than 95 % pure sodium 1,2-di-(9-cis-octadecenoyl)-3-sn-phosphatidyl S-serine (II), and
d) water-soluble excipients suitable for intravenous dosage forms,
wherein the steps indicated in claim 1 are carried out.

7. A process for the preparation of a pharmaceutical composition in the form of a dry preparation, which process comprises dissolving
a) the zinc-phthalocyanine complex and
b) a synthetic, more than 90 % pure phospholipid of formula I, wherein R₁, R₂, Rₐ, R_{b} and R_{c} and also n are as defined, optionally combined with a
c) synthetic, more than 90 % pure phospholipid of formula II, wherein R₃, R₄, Y^{⊕} and n are as defined, in a pharmaceutically acceptable organic solvent, the residual amount of which in a dry preparation is toxicologically harmless, and optionally
d) adding carrier liquid and water-soluble excipients suitable for parenteral dosage forms, and removing the solvent or solvent mixture.

8. A process according to claim 8, wherein a) the zinc-phthalocyanine complex, b) the phospholipid of formula I and optionally c) the phospholipid of formula II are dissolved in piperidine.

## Revendications (Revendications pour l'(les) Etat(s) contractant(s) suivant(s): AT, BE, CH, DE, DK, FR, GB, IT, LI, LU, NL, SE)

1. Compositions pharmaceutiques sous forme de dispersions de liposomes applicables par voie parentérale, contenant
a) le complexe zinc-phtalocyanine,
b) un phospholipide synthétique, pur à plus de 90%, de formule 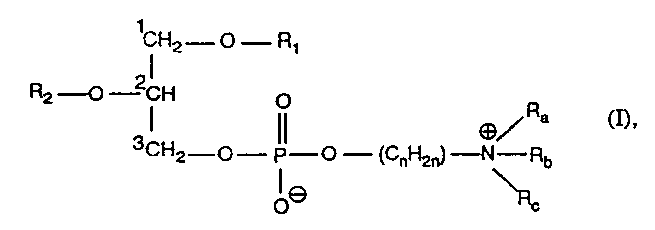 où R₁ représente un groupe alcanoyle en C₁₀-C₂₀ ayant un nombre pair d'atomes de carbone, R₂ est un groupe alcénoyle en C₁₀-C₂₀ ayant un nombre pair d'atomes de carbone, Rₐ, R_{b} et R_{c} sont l'hydrogène ou un groupe alkyle en C₁-C₄ et n est un nombre entier de deux à quatre, éventuellement combinés avec
c) un phospholipide synthétique, pur à plus de 90%, de formule 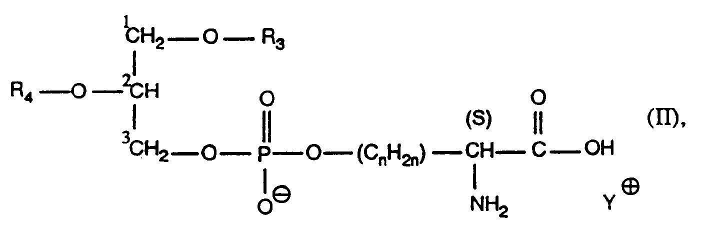 où R₃ et R₄ sont, indépendamment l'un de l'autre, un groupe alcénoyle en C₁₀-C₂₀ ayant un nombre pair d'atomes de carbone, n est un nombre entier de un à trois et Y^{⊕} représente le cation d'une base pharmaceutiquement acceptable, et
d) un liquide vecteur pharmaceutiquement acceptable et éventuellement des adjuvants hydrosolubles appropriés pour des formes de présentation pour voie parentérale.

2. Compositions pharmaceutiques selon la revendication 1 sous forme de dispersions de liposomes applicables par voie intraveineuse, contenant
a) le complexe zinc-phtalocyanine,
b) un phospholipide synthétique de formule I, pur à plus de 95%, dans lequel R₁ représente un groupe n-dodécanoyle, n-tétradécanoyle, n-hexadécanoyle ou n-octadécanoyle et R₂ désigne un groupe 9-cis-dodécénoyle, 9-cis-tétradécénoyle, 9-cis-hexadécénoyle, 9-cis-octadécénoyle ou 9-cis-éicosénoyle, Rₐ, R_{b} et R_{c} sont des groupes méthyle et n vaut deux, éventuellement combiné avec
c) un phospholipide synthétique de formule II, pur à plus de 95%, dans lequel R₃ et R₄ ont des significations identiques et représentent des groupes 9-cis-dodécénoyle, 9-cis-tétradécénoyle, 9-cis-hexadécénoyle, 9-cis-octadécénoyle ou 9-cis-éicosénoyle, n vaut un et Y^{⊕} est l'ion sodium, et
d) un liquide vecteur pharmaceutiquement acceptable et éventuellement des adjuvants hydrosolubles appropriés pour des formes de présentation pour voie intraveineuse.

3. Compositions pharmaceutiques selon la revendication 1 sous forme de dispersions de liposomes applicables par voie intraveineuse, contenant
a) le complexe zinc-phtalocyanine,
b) de la 1-n-hexadécanoyl-2-(9-cis-octadécénoyl)-3-sn-phosphatidylcholine (I) synthétique, pure à plus de 95%, éventuellement combinée avec
c) de la 1,2-di-(9-cis-octadécénoyl)-3-sn-phosphatidyl-S-sérine sodique (II) synthétique, pure à plus de 95%, et
d) un liquide vecteur pharmaceutiquement acceptable et éventuellement des adjuvants hydrosolubles appropriés pour des formes de présentation pour voie intraveineuse.

4. Compositions pharmaceutiques selon la revendication 1 sous forme de dispersions de liposomes applicables par voie intraveineuse, contenant
a) le complexe zinc-phtalocyanine,
b) 70% en poids (par rapport au constituant c)) de 1-n-hexadécanoyl-2-(9-cis-octadécénoyl)-3-sn-phosphatidylcholine (I) synthétique, pure à plus de 95%, combinée avec
c) 30% en poids (par rapport au constituant b)) de 1,2-di-(9-cis-octadécénoyl)-3-sn-phosphatidyl-S-sérine sodique (II) synthétique, pure à plus de 95%, et
d) un liquide vecteur pharmaceutiquement acceptable et éventuellement des adjuvants hydrosolubles appropriés pour des formes de présentation pour voie intraveineuse.

5. Compositions pharmaceutiques sous forme de préparations sèches contenant
a) le complexe zinc-phtalocyanine,
b) un phospholipide synthétique de formule I, pur à plus de 90%, dans lequel R₁, R₂, Rₐ, R_{b} et R_{c}, ainsi que n, ont les significations indiquées dans la revendication 1, éventuellement combiné avec
c) un phospholipide synthétique de formule II, pur à plus de 90%, dans lequel R₃ , R₄, n et Y^{⊕} ont les significations indiquées dans la revendication 1, et éventuellement
d) des adjuvants hydrosolubles, appropriés pour des formes de présentation pour voie intraveineuse.

6. Compositions pharmaceutiques selon la revendication 5 sous forme de lyophilisats contenant
a) le complexe zinc-phtalocyanine,
b) 70% en poids (par rapport au constituant c)) de 1-n-hexadécanoyl-2-(9-cis-octadécénoyl)-3-sn-phosphatidylcholine (I) synthétique, pure à plus de 95%, combinée avec
c) 30% en poids (par rapport au constituant b)) de 1,2-di-(9-cis-octadécénoyl)-3-sn-phosphatidyl-S-sérine sodique (II) synthétique, pure à plus de 95%, ainsi que
d) des adjuvants hydrosolubles appropriés pour des formes de présentation pour voie intraveineuse.

7. Procédé pour la préparation d'une composition pharmaceutique sous forme d'une dispersion de liposomes applicable par voie parentérale, caractérisé en ce que l'on disperse dans une phase aqueuse une préparation sèche contenant
a) le complexe zinc-phtalocyanine,
b) un phospholipide synthétique de formule I, pur à plus de 90%, dans lequel R₁, R₂, Rₐ, R_{b} et R_{c}, ainsi que n, ont les significations indiquées, éventuellement combiné avec
c) un phospholipide synthétique de formule II, pur à plus de 90%, dans lequel R₃ , R₄, n et Y^{⊕} ont les significations indiquées, et éventuellement
d) des adjuvants hydrosolubles, appropriés pour des formes de présentation pour voie parentérale,
et éventuellement on tamponne la solution aqueuse obtenue à pH 7,0-7,8 et/ou on isole une fraction des liposomes ayant un intervalle de diamètres souhaité.

8. Procédé pour la préparation d'une composition pharmaceutique sous forme d'une préparation sèche, caractérisé en ce qu'on dissout
a) le complexe zinc-phtalocyanine et
b) un phospholipide synthétique de formule I, pur à plus de 90%, dans lequel R₁, R₂, Rₐ, R_{b} et R_{c}, ainsi que n, ont les significations indiquées, éventuellement combiné avec
c) un phospholipide synthétique de formule II, pur à plus de 90%, dans lequel R₃ , R₄, n et Y^{⊕} ont les significations indiquées,
dans un solvant organique pharmaceutiquement acceptable, dont la quantité résiduaire dans une préparation sèche est sans risque toxicologique, et éventuellement
d) on ajoute un liquide vecteur et des adjuvants hydrosolubles appropriés pour des formes de présentation pour voie parentérale et on élimine le solvant ou le mélange de solvants.

9. Procédé selon la revendication 8, caractérisé en ce qu'on dissout a) le complexe zinc-phtalocyanine, b) le phospholipide de formule I et éventuellement c) le phospholipide de formule II dans de la pipéridine.

10. Utilisation de compositions pharmaceutiques sous forme de préparations sèches selon la revendication 5 pour la production d'une dispersion de liposomes applicable par voie parentérale.

11. Utilisation du complexe zinc-phtalocyanine, du phospholipide de formule I et éventuellement de formule II pour la production de préparations sèches.

12. Compositions pharmaceutiques selon la revendication 1 pour utilisation dans un procédé pour le traitement thérapeutique du corps humain ou animal.

## Revendications (Revendications pour l'(les) Etat(s) contractant(s) suivant(s): ES, GR)

1. Procédé pour la préparation de compositions pharmaceutiques sous forme de dispersions de liposomes applicables par voie parentérale, contenant
a) le complexe zinc-phtalocyanine,
b) un phospholipide synthétique, pur à plus de 90%, de formule 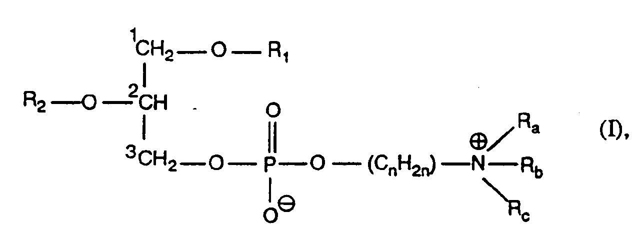 où R₁ représente un groupe alcanoyle en C₁₀-C₂₀ ayant un nombre pair d'atomes de carbone, R₂ est un groupe alcénoyle en C₁₀-C₂₀ ayant un nombre pair d'atomes de carbone, Rₐ, R_{b} et R_{c} sont l'hydrogène ou un groupe alkyle en C₁-C₄ et n est un nombre entier de deux à quatre, éventuellement combinés avec
c) un phospholipide synthétique, pur à plus de 90%, de formule 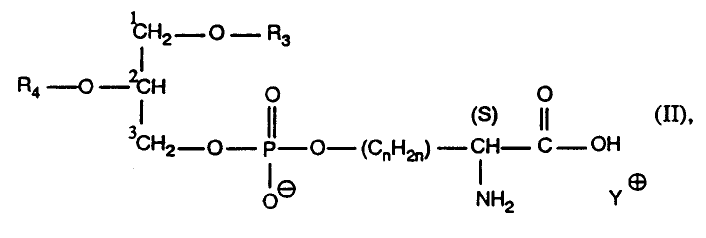 où R₃ et R₄ sont, indépendamment l'un de l'autre, un groupe alcénoyle en C₁₀-C₂₀ ayant un nombre pair d'atomes de carbone, n est un nombre entier de un à trois et Y^{⊕} représente le cation d'une base pharmaceutiquement acceptable, et
d) un liquide vecteur pharmaceutiquement acceptable et éventuellement des adjuvants hydrosolubles appropriés pour des formes de présentation pour voie parentérale,
caractérisé en ce qu'on disperse dans une phase aqueuse une préparation sèche contenant:
a) le complexe zinc-phtalocyanine,
b) un phospholipide synthétique de formule I, pur à plus de 90%, dans lequel R₁, R₂, Rₐ, R_{b} et R_{c}, ainsi que n, ont les significations indiquées, éventuellement combiné avec
c) un phospholipide synthétique de formule II, pur à plus de 90%, dans lequel R₃ , R₄, n et Y^{⊕} ont les significations indiquées, et éventuellement
d) des adjuvants hydrosolubles, appropriés pour des formes de présentation pour voie parentérale,
et éventuellement on tamponne la solution aqueuse obtenue à pH 7,0-7,8 et/ou on isole une fraction des liposomes ayant un intervalle de diamètres souhaité.

2. Procédé pour la préparation de compositions pharmaceutiques selon la revendication 1 sous forme de dispersions de liposomes applicables par voie intraveineuse, contenant
a) le complexe zinc-phtalocyanine,
b) un phospholipide synthétique de formule I, pur à plus de 95%, dans lequel R₁ représente un groupe n-dodécanoyle, n-tétradécanoyle, n-hexadécanoyle ou n-octadécanoyle et R₂ désigne un groupe 9-cis-dodécénoyle, 9-cis-tétradécénoyle, 9-cis-hexadécénoyle, 9-cis-octadécénoyle ou 9-cis-éicosénoyle, Rₐ, R_{b} et R_{c} sont des groupes méthyle et n vaut deux, éventuellement combiné avec
c) un phospholipide synthétique de formule II, pur à plus de 95%, dans lequel R₃ et R₄ ont des significations identiques et représentent des groupes 9-cis-dodécénoyle, 9-cis-tétradécénoyle, 9-cis-hexadécénoyle, 9-cis-octadécénoyle ou 9-cis-éicosénoyle, n vaut un et Y^{⊕} est l'ion sodium, et
d) un liquide vecteur pharmaceutiquement acceptable et éventuellement des adjuvants hydrosolubles appropriés pour des formes de présentation pour voie intraveineuse,
caractérisé en ce qu'on procède aux mesures indiquées dans la revendication 1.

3. Procédé pour la préparation de compositions pharmaceutiques selon la revendication 1 sous forme de dispersions de liposomes applicables par voie intraveineuse, contenant
a) le complexe zinc-phtalocyanine,
b) de la 1-n-hexadécanoyl-2-(9-cis-octadécénoyl)-3-sn-phosphatidylcholine (I) synthétique, pure à plus de 95%, éventuellement combinée avec
c) de la 1,2-di-(9-cis-octadécénoyl)-3-sn-phosphatidyl-S-sérine sodique (II) synthétique, pure à plus de 95%, et
d) un liquide vecteur pharmaceutiquement acceptable et éventuellement des adjuvants hydrosolubles appropriés pour des formes de présentation pour voie intraveineuse,
caractérisé en ce qu'on procède aux mesures indiquées dans la revendication 1.

4. Procédé pour la préparation de compositions pharmaceutiques selon la revendication 1 sous forme de dispersions de liposomes applicables par voie intraveineuse, contenant
a) le complexe zinc-phtalocyanine,
b) 70% en poids (par rapport au constituant c)) de 1-n-hexadécanoyl-2-(9-cis-octadécénoyl)-3-sn-phosphatidylcholine (I) synthétique, pure à plus de 95%, combinée avec
c) 30% en poids (par rapport au constituant b)) de 1,2-di-(9-cis-octadécénoyl)-3-sn-phosphatidyl-S-sérine sodique (II) synthétique, pure à plus de 95%, et
d) un liquide vecteur pharmaceutiquement acceptable et éventuellement des adjuvants hydrosolubles appropriés pour des formes de présentation pour voie intraveineuse,
caractérisé en ce qu'on procède aux mesures indiquées dans la revendication 1.

5. Procédé pour la préparation de compositions pharmaceutiques sous forme de préparations sèches contenant
a) le complexe zinc-phtalocyanine,
b) un phospholipide synthétique de formule I, pur à plus de 90%, dans lequel R₁, R₂, Rₐ, R_{b} et R_{c}, ainsi que n, ont les significations indiquées dans la revendication 1, éventuellement combiné avec
c) un phospholipide synthétique de formule II, pur à plus de 90%, dans lequel R₃ , R₄, n et Y^{⊕} ont les significations indiquées dans la revendication 1, et éventuellement
d) des adjuvants hydrosolubles, appropriés pour des formes de présentation pour voie intraveineuse,
caractérisé en ce qu'on procède aux mesures indiquées dans la revendication 1.

6. Procédé pour la préparation de compositions pharmaceutiques selon la revendication 5 sous forme de lyophilisats contenant
a) le complexe zinc-phtalocyanine,
b) 70% en poids (par rapport au constituant c)) de 1-n-hexadécanoyl-2-(9-cis-octadécénoyl)-3-sn-phosphatidylcholine (I) synthétique, pure à plus de 95%, combinée avec
c) 30% en poids (par rapport au constituant b)) de 1,2-di-(9-cis-octadécénoyl)-3-sn-phosphatidyl-S-sérine sodique (II) synthétique, pure à plus de 95%, ainsi que
d) des adjuvants hydrosolubles appropriés pour des formes de présentation pour voie intraveineuse,
caractérisé en ce qu'on procède aux mesures indiquées dans la revendication 1.

7. Procédé pour la préparation d'une composition pharmaceutique sous forme d'une préparation sèche, caractérisé en ce qu'on dissout
a) le complexe zinc-phtalocyanine et
b) un phospholipide synthétique de formule I, pur à plus de 90%, dans lequel R₁, R₂, Rₐ, R_{b} et R_{c}, ainsi que n, ont les significations indiquées, éventuellement combiné avec
c) un phospholipide synthétique de formule II, pur à plus de 90%, dans lequel R₃ , R₄, n et Y^{⊕} ont les significations indiquées,
dans un solvant organique pharmaceutiquement acceptable, dont la quantité résiduaire dans une préparation sèche est sans risque toxicologique, et éventuellement
d) on ajoute un liquide vecteur et des adjuvants hydrosolubles appropriés pour des formes de présentation pour voie parentérale et on élimine le solvant ou le mélange de solvants.

8. Procédé selon la revendication 7, caractérisé en ce qu'on dissout a) le complexe zinc-phtalocyanine, b) le phospholipide de formule I et éventuellement c) le phospholipide de formule II dans de la pipéridine.
